# EUROPÄISCHE PATENTSCHRIFT

(11) **EP 3 261 684 B1**
(45) Veröffentlichungstag und Bekanntmachung des Hinweises auf die Patenterteilung: **14.08.2019**
(21) Anmeldenummer: 16703914.8
(22) Anmeldetag: 03.02.2016
(51) Int. Cl.: A61L 27/32, A61L 27/34, A61L 27/46

(54) **MATERIAL FÜR KNOCHENIMPLANTATE UND VERFAHREN ZU DESSEN HERSTELLUNG**
MATERIAL FOR BONE IMPLANTS, AND A METHOD FOR PRODUCING SAME
MATÉRIAU POUR IMPLANT OSSEUX ET PROCÉDÉ POUR LE PRODUIRE

(30) Priorität: 24.02.2015 DE 102015002398
(43) Veröffentlichungstag der Anmeldung: 03.01.2018
(73) Patentinhaber: Universität Konstanz, 78464 Konstanz (DE)
(72) Erfinder: CÖLFEN, Helmut, 78465 Konstanz-Dingelsdorf (DE); TIAN, Liangfei, BS8 1JL, Bristol (GB); KNAUS, Jennifer, 78467 Konstanz (DE)
(74) Vertreter: Müller-Boré & Partner Patentanwälte PartG mbB
(86) Internationale Anmeldenummer: PCT/EP2016/000174
(87) Internationale Veröffentlichungsnummer: WO 2016/134819

(56) Entgegenhaltungen:
- EP-A2- 1 693 074
- S. MUNISAMY ET AL: "A Bone-Like Precoating Strategy for Implants: Collagen Immobilization and Its Mineralization on Pure Titanium Implant Surface", JOURNAL OF ORAL IMPLANTOLOGY, Bd. 34, Nr. 2, 1. April 2008 (2008-04-01), Seiten 67-75, XP055265318, US ISSN: 0160-6972, DOI: 10.1563/1548-1336(2008)34[67:ABPSFI]2.0.CO ;2
- HAIYONG AO ET AL: "Improved hMSC functions on titanium coatings by type I collagen immobilization", JOURNAL OF BIOMEDICAL MATERIALS RESEARCH. PART A, Bd. 102, Nr. 1, 10. Mai 2013 (2013-05-10), Seiten 204-214, XP055265416, HOBOKEN, NY, US ISSN: 1549-3296, DOI: 10.1002/jbm.a.34682
- DATABASE WPI Week 200935 Thomson Scientific, London, GB; AN 2009-J56455 XP002756529, & KR 2009 0043857 A (UNIV YEUNGNAM IND ACADEMIC COOP FOUND) 7. Mai 2009 (2009-05-07)

## Beschreibung

Die vorliegende Erfindung betrifft ein Material für Knochenimplantate, umfassend eine Oberfläche aus oxidischen Keramikmaterialien oder Polyetheretherketon (PEEK), eine kovalent an diese Oberfläche gebundene Matrix aus Kollagen oder Gelatine, sowie in diese Matrix eingelagertes Calciumphosphat. Die vorliegende Erfindung betrifft weiter ein Verfahren zur Herstellung des erfindungsgemäßen Materials, Knochenimplantate, umfassend das erfindungsgemäße Material.

In den letzten Jahren nahm die Anzahl der Patienten, die an Knochenschäden leiden und/oder ein Knochenimplantat benötigen, tendenziell zu. Dieser Trend betont die Notwendigkeit, hochwertige, stabile und funktionelle Knochenersatzmaterialien zu erforschen.

Die Anforderungen an hochwertige und funktionsgerechte Knochenimplantate sind vielfältig und es ist schwierig, alle Anforderungen mit einem Material zu erfüllen. Die Funktionalität eines Implantatmaterials ist dabei schwer vorherzusagen, da der natürliche Prozess der Knochenwundheilung und Implantat-Einheilung sehr komplex und zum Teil noch nicht vollständig verstanden ist.

Die Wundheilung von harten oder weichen Geweben nach einem operativen Eingriff, wie beispielsweise einer Knochenimplantation, umfasst viele zelluläre und extrazelluläre Ereignisse. Der Heilungsprozess an der Kontaktfläche zwischen dem Knochenimplantat und dem Knochen umfasst vier Stufen, welche teilweise ineinander überlappend stattfinden. Darunter fallen Entzündungsreaktionen, die Bildung eines weichen Kallus, gefolgt von der Bildung eines harten Kallus und schließlich die Remodellierungsphase.

Nach einem operativen Eingriff adsorbieren zunächst Proteine und andere Moleküle des Blutes und der Gewebsflüssigkeiten an der Oberfläche des Implantats. Wird vaskularisiertem Gewebe eine Wunde zugefügt, führt dies nicht nur zu Entzündungsreaktionen, sondern auch zur Aktivierung einer Vielzahl weiterer körpereigener Schutzsysteme, wie beispielsweise des extrinsischen und intrinsischen Koagulationssystems, des Komplementsystems, des fibrinolytischen Systems und des Kininsystems. Hiernach folgen zwei sequentiell ablaufende Phasen, die ebenfalls ineinander übergehend verlaufen können, nämlich die akute und die chronische Entzündungsreaktion. Das Blut koaguliert zu einem Blutgerinnsel, welches aus Fibrin als Hauptbestandteil aufgebaut ist. Parallel hierzu werden Zytokine und weitere Wachstumsfaktoren freigesetzt, um weiße Blutzellen zur Wunde zu rekrutieren. Hierbei werden in der akuten Entzündungsantwort Neutrophile und mononukleäre Zellen wie beispielsweise Monozyten rekrutiert. Mononukleäre Zellen differenzieren sich zu Makrophagen und lagern sich an der Oberfläche des Implantats an. Bei einer normal verlaufenden Wundheilung sind Makrophagen dafür verantwortlich, die Wunde von Bakterien, Zelltrümmern und anderen Verunreinigungen via Phagozytose zu reinigen. Das Implantatmaterial wird dabei vom Körper ebenfalls als Fremdkörper empfunden. Da das Implantat allerdings wesentlich größer als die Makrophagen ist, können diese das Material nicht phagozytieren. Diese Ereignisse führen schließlich zu der Phase der chronischen Entzündung an der Material/Gewebsgrenze. Dabei fusionieren die Makrophagen und bilden mehrkernige Riesenzellen, um den Fremdkörper zu umschließen. Die Makrophagen sorgen ebenfalls für die Rekrutierung weiterer Zellen wie beispielsweise Fibroblasten, welche fibröse Gewebe an der Oberfläche des Implantats ablagern.

Nach der Entzündungsphase bildet sich ein weicher Kallus. Dieser besteht aus Knochenvorläuferzellen und Fibroblasten, welche sich in einer ungeordneten Matrix aus nicht-kollagenen Proteinen und Kollagen befinden. Diese Matrix wird graduell von besagten Zellen als erste Reaktion gebildet und ist strukturell dem Geflechtknochen ähnlich. Der weiche Kallus wird schließlich graduell von Osteoblasten in geordnete lamellare Knochenstruktur umgebaut. Osteoblasten sekretieren dabei Typ-I-Kollagen, Calciumphosphat und Calciumcarbonat mit einer willkürlichen, zufälligen Orientierung. Die Remodellierungsphase überlappt mit der Bildung des harten Kallus. Dies geschieht durch Resorption der ungeordneten Knochenstrukturen durch Osteoclasten und anschließender Bildung von geordneten Knochenstrukturen durch Osteoblasten.

Primär sollte ein Implantatmaterial nicht toxisch sein und zudem *in vivo* keine Entzündungs-, Immun- oder anderen negativen oder ungünstigen Reaktionen hervorrufen. Wenn sich einzelne Partikel vom Implantat lösen, dürfen diese ebenfalls keine der vorher genannten Reaktionen auslösen und sollten zudem entweder im Körper abbaubar oder sekretierbar sein, um eine permanente Anhäufung und Anlagerung im Körper oder eine aseptische Endoprothesenlockerung zu vermeiden. Potentielle Knochenmaterialien sollten daher eine zuverlässige Stärke, hohe Widerstandsfähigkeit, hohe Abriebsfestigkeit, Korrosionsbeständigkeit, sowie eine dem Knochen ähnliche Steifheit besitzen. Letztgenanntes spielt im Speziellen im Kontext des sogenannten "stress shielding" eine große Rolle. Der Knochen ist ein dynamisches System, welches je nach mechanischer Beanspruchung ab- oder aufgebaut wird. Wird nun ein Knochenimplantatmaterial eingesetzt, das eine im Vergleich zur Knochensubstanz höhere Steifheit besitzt, übernimmt dieses einen Großteil der mechanischen Belastung, wodurch der umgebende Knochen nach und nach abgebaut wird.

Ein weiteres Kriterium ist die sogenannte Biokompatibilität. Ein potentielles Implantatmaterial sollte entweder möglichst bioinert oder bioaktiv sein. Ein bioinertes Material verursacht keinerlei chemische oder biologische Reaktionen im Körper. Ein bioaktives Material hingegen fördert ein schnelles Einwachsen des Implantats in das umgebende Gewebe und sorgt so für eine schnelle und langanhaltende Fixierung des Implantats im Körper. Dieser Effekt wird als sogenannte "Osseointegration" bezeichnet. Diese Fähigkeit eines Biomaterials, die Zelladhäsion und -migration zu fördern ist entscheidend für die frühen Phasen der Wundheilung und die späteren Phasen der Knochenneubildung und hängt stark vom initialen Kontakt zwischen den Zellen und dem Implantatmaterial ab.

Im medizinischen Alltag sind autologe Knochenimplantate der "Goldstandard". Die Verwendung von autologem Knochenmaterial des Patienten hat allerdings einige Nachteile, welche die Anwendung dieses Ansatzes limitieren. Für größere Knochendefekte wird viel Knochenmaterial benötigt, welches häufig nicht zur Verfügung steht. Ein weiterer Nachteil ist, dass für die Gewinnung von autologem Knochenmaterial ein zweiter operativer Eingriff benötigt wird. Daher entstand in den letzten Jahren ein großer Bereich, welcher sich mit der Erforschung neuer oder der Verbesserung bereits vorhandener Knochenimplantatmaterialien beschäftigt. Es existiert eine breite Palette von unterschiedlichen halb- oder vollsynthetischen Materialien, wie zum Beispiel Keramiken, Knochenzemente, Polymere, Metalle oder Kompositmaterialien, welche zum Teil bereits im medizinischen Alltag Anwendung finden. Jedes dieser Materialien hat vorteilhafte Eigenschaften, jedoch liegt bei allen noch ein großes Verbesserungspotential. Ein inhärentes Problem der Keramiken und Knochenzemente ist beispielsweise deren Brüchigkeit, welches zu mechanischem Versagen des Implantats und manchmal sogar zu Problemen beim biologischen Abbau der Bruchstücke des Implantats führt, was schließlich zu einer aseptischen Prothesenlockerung führen kann. Ein inhärentes Problem bei metallbasierten Materialien ist deren Undurchlässigkeit für Röntgenstrahlen. Dies ist im Bereich der bildgebenden medizinischen Diagnostik nachteilig. Ebenfalls ist deren hohe Steifheit von Nachteil, die zu den bereits beschriebenen Problemen führen kann. Des Weiteren können Metalle aufgrund ihrer chemischen Natur nicht als biomimetische Materialien bezeichnet werden. Auch polymerbasierte Materialien, wie beispielsweise Polyethylen, Polystyrol oder Polyetheretherketon (PEEK) wurden vorgeschlagen.

Im Bereich der Oberflächenmodifikationen von Knochenimplantaten finden Beschichtungen mit verschiedenen Calciumphosphaten, wie beispielsweise Hydroxylapatit oder Tricalciumphosphat, eine weite Verbreitung. Die exakte chemische Struktur von biologischen Apatit ist sehr komplex und kann generell als ein mit Carbonationen nicht-stöchiometrisch substituierter, calciumdefizienter Hydroxylapatit angesehen werden. Stöchiometrisch reiner Hydroxylapatit ist eine Form des Apatit mit der chemischen Zusammensetzung Ca₅(PO₄)₃(OH) und wird üblicherweise als Ca₁₀(PO₄)₆(OH)₂ notiert, um zu verdeutlichen, dass die Elementarzelle zwei Einheiten enthält. Die natürliche Biomineralisation ist ein komplexer, mehrstufiger Prozess, dessen Mechanismus noch nicht vollständig geklärt ist. Der Knochen besteht zum einen aus der Mineralphase, zum anderen aber auch zu 30 bis 40% aus einer Proteinmatrix, welche die Knochenmatrix aufbaut. Die organische Knochenmatrix besteht wiederum aus mehreren Komponenten. 95% bestehen dabei aus Kollagen. Die restlichen 5% der organischen Matrix bestehen hauptsächlich aus Proteoglykanen und anderen adhäsiven Glykoproteinen.

Das Kollagen des Knochens höherer Vertebraten besteht größtenteils aus Kollagen vom Typ I. Kollagen gehört zu einer Gruppe Proteine, welche bis zu 25 bis 30% der gesamten Proteinmenge des menschlichen Körpers ausmacht. Bis dato wurden 28 unterschiedliche Kollagentypen in Vertebraten identifiziert, wobei der Typ I mit einer Häufigkeit von 90% der Gesamtkollagenmenge am häufigsten vorkommt. Typ-I-Kollagen hat die Fähigkeit, sich in Fibrillen anzuordnen. Typischerweise ordnen sich Kollagenmonomereinheiten in einer einheitlichen rechtshändigen Tripelhelix an, welche üblicherweise aus zwei identischen Polypeptidketten (α₁) besteht, welche wiederum aus etwa 334 Wiederholungseinheiten einer (Gly-X-Y)-Sequenz aufgebaut sind, und einer weiteren Kette (α₂), welche leicht in ihrer chemischen Zusammensetzung variiert. Hierbei belegt die Aminosäure Prolin häufig die X-Position und Hydroxyprolin sehr häufig die Y-Position der Wiederholeinheit.

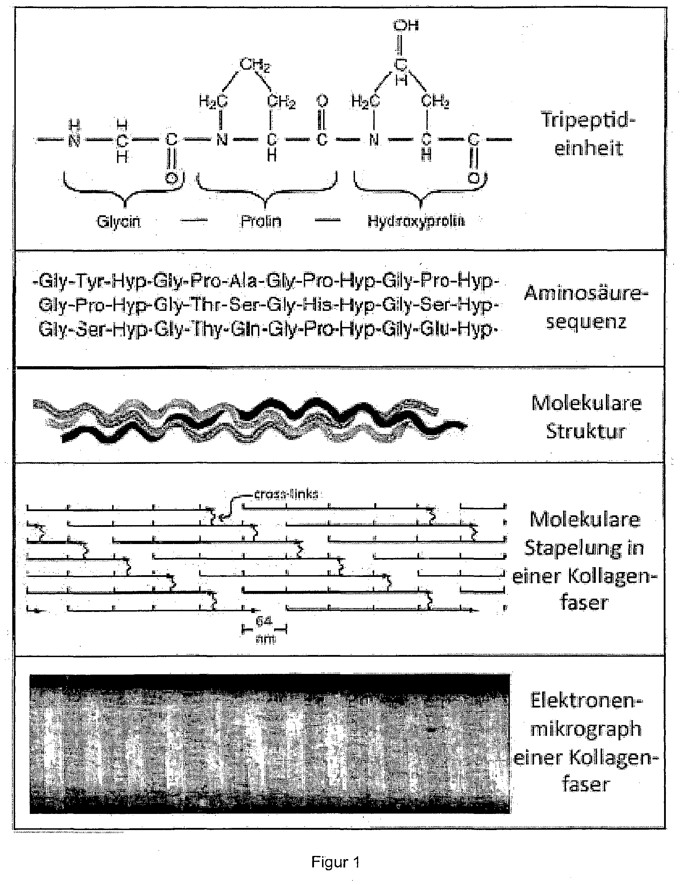

Die genaue Aminosäurezusammensetzung variiert mit Typ und Herkunft des Kollagens. Jede alpha-Kette windet sich in einer linksgängigen Helix mit etwa drei Aminosäuren pro Drehung, wobei die Kette über Wasserstoffbrückenbindungen stabilisiert wird. Durch laterale Aggregation können die Tripelhelices stabile Fasern bilden, wie sie beispielsweise in Sehnen auftreten. Dabei sind die Monomereinheiten leicht versetzt und durch eine Lücke von etwa 64 bis 67 nm voneinander getrennt. Durch eine Aldolkondensation zwischen Hydroxylysinresten kommt es zu einer kovalenten Vernetzung der Fibrillen, welche weitere Stabilität, Reiß- und Zugfestigkeit liefern. Die Kollagenmonomereinheiten sind etwa 300 nm lang und 1,5 nm im Durchmesser. Der strukturelle Aufbau von Kollagenfasern ist in Figur 1 gezeigt.

Eine Änderung nur einer Aminosäure der charakteristischen Wiederholeinheit hat große Auswirkung auf die Stabilität der gesamten Struktur. Dies wird beispielsweise in der Krankheit *Osteogenesis imperfecta* deutlich, bei der ein Glycin-Rest durch andere Aminosäuren ausgetauscht ist. Die Kollagenketten verlieren so die Fähigkeit, stabile Fasern zu bilden, was im Endeffekt in sehr brüchigen Knochen des Patienten resultiert. Kollagen kann aus Geweben wie beispielsweise Kalbshaut oder Bändern und Sehnen aus Rattenschwänzen isoliert werden.

Kollagen enthält als Teil der extrazellulären Matrix ebenfalls zelluläre Erkennungssquenzen in seiner Aminosäuresequenz, die der zellulären Adhäsion diverser Zelltypen zu dienen. Ein Beispiel einer solchen Sequenz ist das sog. Arginin-Glycin-Asparaginsäure (RGD) -Motiv. Dieses kommt außer in Kollagen ebenfalls in Fibronectinen, Vitronectinen, Fibrinogenen und Lamininen vor. In nativen, fibrillären Kollagen ist diese natürlich vorkommende Sequenz durch die üblicherweise tripelhelikale Struktur des Kollagens verdeckt. Durch Denaturierung der Kollagenhelices können diese RGD-Sequenzen allerdings exponiert werden und sind so den Zellen zugänglich. Es wird vermutet, dass durch dieses Freiwerden der Sequenzen bei einer Verletzung die Wundheilung durch verbesserte Adhäsion der verantwortlichen Zelltypen gefördert werden soll.

Bei der Biomineralisation von nativem Kollagen kann eine typische Abfolge beobachtet werden, wobei die Mineralisation scheinbar durch weitere extrazelluläre Matrixproteine gesteuert wird. Diese sind in parallelen Strängen entlang der Kollagenfaser gebunden. Die Proteine scheinen die amorphen Vorstufen des Calciumphosphats (amorphes Calciumorthophosphat, ACP) zu stabilisieren und die daran anschließende Nukleation und das Wachstum des Apatit in der Spaltzone zwischen den Kollagenmonomeren zu steuern. Während der Knochenbiomineralisation werden dabei primär Partikel im Nanometerbereich gebildet, welche dann schnell in der Länge wachsen und mit benachbarten Nanopartikeln verschmelzen, um schließlich nadelförmige Strukturen auszubilden. Diese Wachstumsprozesse verursachen einen partiellen Bruch der Bindungen zwischen den einzelnen Fasern. Schließlich verschmelzen die nadelförmigen Strukturen mit benachbarten Kristallen und bilden dabei plättchenförmige Strukturen aus, welche in die [0 0 1]-Richtung entlang der c-Achse der Kollagenfaser wachsen. Sie besitzen also eine uniaxiale Orientierung und sind zudem kohärent entlang der ab-Ebene zu parallelen Stapeln aufgebaut.

Häufig werden für den Prozess der Knochenmineralisation drei Verbindungen als intermediäre Phasen diskutiert. Das bereits erwähnte amorphe Calciumphosphat (ACP), Octacalciumphosphat (OCP) und Dicalciumphosphat-Dihydrat (DCPD, das Mineral Brushit) Synthetisches ACP ist auch häufig ein Zwischenprodukt bei der Fällung von Calciumphosphaten aus wässrigen Lösung, wobei seine chemische Zusammensetzung stark von den Fällungsbedingungen abhängt. Die Struktur von ACP ist noch nicht eindeutig bestimmt und erscheint in einem Röntgendiffraktogramm amorph. Auf Grundlage von röntgenabsorptionsspektroskopischen Messungen wird ebenfalls diskutiert, dass ACP eine apatitische Mikrostruktur mit so kleinen Domänengrößen aufweist, dass es röntgenamporph erscheint.

Stöchiometrisch reines, synthetisches OCP besitzt die chemische Zusammensetzung Ca₈(HPO₄)₂(PO₄)₄ · 5 H₂O) und tritt ebenfalls häufig als metastabiles Zwischenprodukt bei der Fällung von Calciumphosphaten aus wässriger Lösung auf. Aus strukturellem Gesichtspunkten besteht es aus apatitähnlichen Schichten, die durch hydratisierte Schichten getrennt sind. Stöchiometrisch reines, synthetisches DCPD besitzt die chemische Zusammensetzung CaHPO₄ · 2 H₂O und kann als metastabiles Zwischenprodukt ebenfalls bei der Fällung von Calciumphosphaten aus wässriger Lösung kristallisiert werden. Es kann bei Temperaturen über 80 °C leicht durch Entwässerung in Dicalciumphosphat-Anhydrat überführt werden.

Knochenimplantatmaterialien wurden im Stand der Technik durch verschiedene chemische oder physikalische Verfahren mit Hydroxylapatit oder Tricalciumphosphat beschichtet. Diese Calciumphosphate werden meist durch einfache chemische Verfahren mittels Präzipitation aus wässrigen Lösungen hergestellt. Das Aufbringen auf die Oberfläche des Implantatmaterials erfolgt dabei entweder direkt aus der Lösung auf die Oberfläche oder mittels physikalischer Methoden wie "electrospray deposition". Allerdings ist bei der Beschichtung von Materialien mit Apatit beispielsweise sowohl die geringe Adhäsion der Calciumphosphate auf dem Implantat nachteilig, als auch deren limitierter Zusammenhalt innerhalb der einzelnen Calciumphosphatlagen. Mit diesen Verfahren sollte eine dem Knochen möglichst ähnliche Struktur auf der Oberfläche generiert werden, um das Einheilen des Materials in den Knochen zu begünstigen. Hierbei wird allerdings außer Acht gelassen, dass der Knochen selbst ein stark hierarchisch aufgebautes Kompositmaterial ist, welches aus einer Matrix und einer Mineralphase besteht. Einige Verfahren zur Beschichtung von Implantatmaterialien mit Kollagen wurden auf ihre *in vivo* Funktionalität untersucht. Häufig wurden hierbei Kollagen-beschichtete Titanimplantate wie Schrauben oder Nägel in verschiedenen *in vivo*-Systemen analysiert. Dabei wurde beispielsweise von positiven Effekten bezüglich des Einwachsens des Materials in den umgebenden Knochen sowie der Knochenneubildung berichtet. Allerdings finden sich im Stand der Technik widersprüchliche Ergebnisse bezüglich kollagenbeschichteter Titanimplantate. So konnte beispielsweise keine verbesserte Osseointegration von kollagenbeschichteten porösen Titanzylindern, welche in die Diaphyse von Schaftibiae implantiert wurden, festgestellt werden.

Ebenfalls wurde im Stand der Technik von Verfahren zur kovalenten oder nichtkovalenten Immobilisierung diverser Proteine der extrazellulären Matrix, wie beispielsweise Fibronectin oder kurzen Peptiden, auf Oberflächen berichtet. Diese zeigten zum Teil in *in vitro* Testsystemen positive Effekte auf die Zelladhäsion und Proliferation.

Häufig wird Kollagen aus Kosten- und Handhabungsgründen durch dessen denaturierte Form Gelatine ersetzt. Die Herstellung der Gelatine geschieht üblicherweise durch physikalischen und chemischen Abbau oder thermische Denaturierung von nativem Kollagen. Im Gegensatz zu nativem Kollagen ist Gelatine bei einem physiologischen pH-Wert wasserlöslich und schmilzt bei einer Sol-Gel-Übergangstemperatur von 25 bis 30°C. Nach Abkühlen entstehen durchsichtige Gele. Im Stand der Technik wird ebenfalls die nicht-kovalente Aufbringung von Gelatine auf Oberflächen von arteriellen Implantatmaterialien berichtet. S.Munisamy et al. (Journal of Oral Implantology; 34(2), 01.04.2008; 67-75; XP055265318) beschreiben Titan-Knochenimplantate mit mechanisch oder mit Säure aufgerauhter Oberfläche, auf die eine Kollagenschicht aufgebracht ist. Eine Mineralsierung mit einer Calciumsalzlösung führt zur Präzipitierung von Calciumphosphat.

EP1693074 beschreibt Metall-Knochenimplantate die mit Keramikschichten und daran kovalent gebundenem Kollagen versehen sind. Als Keramik ist Hydroxyapatit genannt.

Auf dieser Grundlage liegt der vorliegenden Erfindung die Aufgabe zugrunde, ein Material für Knochenimplantate bereitzustellen, welches knochenähnliche Strukturen aufweist, die sowohl die Protein- als auch die Mineralphase des natürlichen Knochens beinhalten. Zudem soll ein entsprechendes Herstellungsverfahren bereitgestellt werden.

Diese Aufgabe wird durch die in den Ansprüchen gekennzeichneten Ausführungsformen gelöst.

Insbesondere betrifft ein Gegenstand der vorliegenden Erfindung ein Material für Knochenimplantate, umfassend:
(a) eine Oberfläche, umfassend ein Material, ausgewählt aus der Gruppe, bestehend aus oxidischen Keramikmaterialien und Polyetheretherketon (PEEK),
(b) eine kovalent an diese Oberfläche gebundene Matrix, umfassend Kollagen und/oder Gelatine, und
(c) in diese Matrix eingelagertes Calciumphosphat.

Die Begriffe "Material für Knochenimplantate" und "Knochenimplantatmaterial" werden hierin synonym gebraucht. Das erfindungsgemäße Material für Knochenimplantate weist bioaktive Eigenschaften auf. Der hierin verwendete Begriff "bioaktiv" bezeichnet die Eigenschaft des erfindungsgemäßen Materials für Knochenimplantate, ein schnelles Einwachsen in das umgebende Gewebe zu ermöglichen und so für eine schnelle und langanhaltende Fixierung des Implantats im Körper zu sorgen. Diese Eigenschaft ergibt sich alleine aus den in den vorstehenden Unterpunkten (a) bis (c) definierten technischen Merkmalen.

Das erfindungsgemäße Material für Knochenimplantate wird auf feste Materialien oder Körper aufgebracht, welche als Knochenimplantat Verwendung finden. Diese Körper können jede beliebige gewünschte oder erforderliche dreidimensionale Gestalt aufweisen. Bevorzugt umfasst die gesamte Oberfläche des erfindungsgemäßen Materials für Knochenimplantate das im vorstehenden Unterpunkt (a) definierte Material oder besteht aus diesem. Geeignete Materialien, auf welche das erfindungsgemäße Material aufgebracht werden kann, können hierbei aus im Stand der Technik bekannten Keramikmaterialien, Metallen, Polymeren, Kompositmaterialien oder Kombinationen davon ausgewählt sein.

Die Oberfläche des erfindungsgemäßen Materials für Knochenimplantate umfasst ein Material, ausgewählt aus der Gruppe, bestehend aus oxidischen Keramikmaterialien und Polyetheretherketon (PEEK), oder besteht aus diesem. Geeignete oxidische Keramikmaterialien sind im Stand der Technik bekannt. In einer bevorzugten Ausführungsform ist das Material PEEK. Dieses Material ist mechanisch nativem Knochenmaterial sehr ähnlich und so als Knochenimplantatmaterial gut geeignet.

Das erfindungsgemäße Material für Knochenimplantate umfasst eine kovalent an die Oberfläche gebundene Matrix, umfassend Kollagen, bevorzugt Typ-I-Kollagen, und/oder Gelatine. In bestimmten Ausführungsformen besteht diese Matrix aus Kollagen, bevorzugt Typ-I-Kollagen, und/oder Gelatine. Gelatine ist eine denaturierte Form von Kollagen und ist im Vergleich zu diesem kostengünstiger und einfacher handzuhaben. Daher ist die Verwendung von Gelatine als Matrixmaterial bevorzugt. Verfahren zur kovalenten Bindung einer Matrix aus Kollagen und/oder Gelatine an eine Oberfläche sind nachfolgend beschrieben. Die kovalent gebundene Matrix weist typischerweise eine Schichtdicke von 100 bis 150 nm auf, kann aber auch dicker sein oder dünner sein. Insbesondere kann die kovalent gebundene Matrix eine Schichtdicke von 1 nm bis 10 µm, bevorzugt von 10 nm bis 1 µm, mehr bevorzugt von 20 nm bis 500 nm, mehr bevorzugt von 30 nm bis 300 nm, mehr bevorzugt von 50 nm bis 200 nm und am meisten bevorzugt von 100 bis 150 nm aufweisen. Weiter bedeckt die kovalent gebundene Matrix bevorzugt die gesamte Oberfläche des erfindungsgemäßen Materials für Knochenimplantate.

Schließlich umfasst das erfindungsgemäße Material für Knochenimplantate in die genannte Matrix eingelagertes Calciumphosphat, bevorzugt Calciumorthophosphat in allen mineralischen Formen, besonders bevorzugt ausgewählt aus der Gruppe, bestehend aus amorphem Calciumorthophosphat (ACP), Dicalciumphophat-Dihydrat (DCPD; Brushit), Octacalciumphosphat und Hydroxylapatit, auch mit partieller Fluorid-, Chlorid- oder Carbonatsubstitution, wobei ACP, Hydroxylapatit und Octacalciumphosphat besonders bevorzugt sind. Verfahren zum Einlagern der genannten Calciumphosphate in eine entsprechende Matrix sind nachfolgend beschrieben.

In einer bevorzugten Ausführungsform umfasst das erfindungsgemäße Material für Knochenimplantate PEEK oder besteht aus diesem, wobei das Kollagen und/oder die Gelatine der kovalent gebundenen Matrix über einen Linker, ausgewählt aus der Gruppe, bestehend aus einem Dicarbonsäure-Linker, einem Maleimid-Linker und einem Hexamethylendiisocyanat-Linker, an das PEEK gebunden ist. Entsprechende Linker sind im Stand der Technik bekannt. Verfahren für die kovalente Bindung von Kollagen und/oder Gelatine an PEEK sind nachfolgend beschrieben.

In anderen Ausführungsformen umfasst das erfindungsgemäße Material für Knochenimplantate oxidische Keramikmaterialien, oder besteht aus diesen, wobei das Kollagen und/oder die Gelatine der kovalent gebundenen Matrix über einen Silanlinker gebunden sind. Geeignete Silanlinker und entsprechende Verfahren zur Bindung von Kollagen und/oder Gelatine sind im Stand der Technik bekannt.

In einer besonders bevorzugten Ausführungsform betrifft die vorliegende Erfindung ein Material für Knochenimplantate, umfassend:
(a) eine Oberfläche, bestehend aus PEEK,
(b) eine kovalent an diese Oberfläche gebundene Matrix, bestehend aus Gelatine, und
(c) in diese Matrix eingelagerten Hydroxylapatit.

Ein weiterer Gegenstand der vorliegenden Erfindung betrifft ein Verfahren zur Herstellung eines erfindungsgemäßen Materials für Knochenimplantate, umfassend die Schritte:
(a) Bereitstellen einer Oberfläche, umfassend ein Material, ausgewählt aus der Gruppe, bestehend aus oxidischen Keramikmaterialien und Polyetheretherketon (PEEK),
(b) kovalentes Ankoppeln einer Matrix, umfassend Kollagen und/oder Gelatine, an diese Oberfläche, und
(c) Mineralisieren der Matrix mit Calciumphosphat.

Für diesen Gegenstand der vorliegenden Erfindung gelten alle relevanten Definitionen und bevorzugten Ausführungsformen, die vorstehend für das erfindungsgemäße Material für Knochenimplantate aufgeführt wurden, in analoger Weise.

Verfahren zur kovalenten Ankopplung einer Kollagen und/oder Gelatine umfassenden Matrix an eine Oberfläche gemäß Schritt (b) des erfindungsgemäßen Verfahrens unterliegen keinen besonderen Beschränkungen und sind im Stand der Technik bekannt.

In einer bevorzugten Ausführungsform umfasst die Oberfläche PEEK oder besteht aus diesem, und Schritt (b) des erfindungsgemäßen Verfahrens umfasst die Schritte:
(b1) Aktivieren der Oberfläche durch Reduktion der Ketogruppe des PEEK zu einer Hydroxylgruppe,
(b2) kovalentes Ankoppeln eines Linkermoleküls, ausgewählt aus der Gruppe, bestehend aus einem Dicarbonsäure-Linker, einem Maleimid-Linker und einem Hexamethylendiisocyanat-Linker, an diese aktivierte Oberfläche, und
(b3) kovalentes Ankoppeln des Kollagens und/oder der Gelatine an das Linkermolekül.

Verfahren zur Reduktion der Ketogruppe von PEEK zu einer Hydroxylgruppe unterliegen keinen besonderen Beschränkungen und umfassen beispielsweise das Inkubieren der Oberfläche mit einer Lösung aus Natriumborhydrid und Dimethylsulfoxid oder einer Lösung aus Lithiumaluminiumhydrid in organischen Lösungsmitteln.

Verfahren zur Ankopplung von Linkermolekülen an eine entsprechend aktivierte PEEK-Oberfläche unterliegen ebenfalls keinen besonderen Beschränkungen. In Fällen, in denen das Linkermolekül ein Dicarbonsäure-Linker ist, umfassen sie beispielsweise das Inkubieren der Oberfläche mit einer Lösung, enthaltend die entsprechende Dicarbonsäure, N,N'-Dicyclohexylcarbodiimid (DCC) und 4-Dimethylaminopyridin (DMAP) in Tetrahydrofuran (THF). In Fällen, in denen das Linkermolekül ein Maleimid-Linker ist, umfassen sie beispielsweise das Inkubieren der Oberfläche mit einer Lösung, enthaltend Triphenylphosphan, Azodicarbonsäurediethylester und Maleimid in THF. In Fällen, in denen das Linkermolekül ein Hexamethylendiisocyanat-Linker ist, umfassen sie beispielsweise das Inkubieren der Oberfläche unter Schutzgasatmosphäre und trockenen Reaktionsbedingungen mit einer Lösung, enthaltend Hexamethylendiisocyanat und katalytische Mengen von 1,4-Diazabicyclo[2.2.2]octan (DABCO) in Toluol.

Verfahren zur Ankopplung von Kollagen und/oder Gelatine an entsprechende Linkermoleküle unterliegen keinen besonderen Beschränkungen und sind im Stand der Technik bekannt. Ist das Linkermolekül beispielsweise eine Dicarbonsäure, umfasst Schritt (b3) des erfindungsgemäßen Verfahrens das Inkubieren der Oberfläche mit einer 1-Ethyl-3-(3-dimethylaminopropyl)carbodiimid (EDC)/N-Hydroxysuccinimid (NHS)-Lösung (EDC/NHS-Lösung) und nachfolgend das Inkubieren der Oberfläche mit einer Kollagen und/oder Gelatine enthaltenden Lösung. Ist das Linkermolekül ein Maleimid-Linker oder ein Hexamethylendiisocyanat-Linker, umfasst Schritt (b3) des erfindungsgemäßen Verfahrens das Inkubieren der Oberfläche mit einer Kollagen und/oder Gelatine enthaltenden Lösung.

Verfahren zum Mineralisieren einer Kollagen und/oder Gelatine enthaltenden Matrix mit Calciumphosphaten gemäß Schritt (c) des erfindungsgemäßen Verfahrens unterliegen keinen besonderen Beschränkungen. Sie umfassen für den Fall, dass ACP verwendet wird, beispielsweise das Inkubieren der Oberfläche mit einer Lösung, umfassend Calciumchlorid, Dikaliumhydrogenphosphat und einen Nukleationsinhibitor. Dieser Nukleationsinhibitor ist bevorzugt ein nicht-kollagenes Protein oder Proteinanalogon, besonders bevorzugt poly-Asparaginsäure und/oder Fetuin. Für den Fall, dass Hydroxylapatit verwendet wird, umfassen Sie beispielsweise das Inkubieren der Oberfläche mit einer Lösung, umfassend Calciumchlorid und Dikaliumhydrogenphosphat.

Ein weiterer Gegenstand der vorliegenden Erfindung betrifft ein Knochenimplantat, umfassend ein festes Material und/oder einen festen Körper, auf das/den das erfindungsgemäße Knochenimplantatmaterial aufgebracht ist.

Für diesen Gegenstand der vorliegenden Erfindung gelten alle relevanten Definitionen und bevorzugten Ausführungsformen, die vorstehend für das erfindungsgemäße Material für Knochenimplantate aufgeführt wurden, in analoger Weise.

Implantate wachsen in den menschlichen Körper umso besser ein, und werden um so stabiler mit dem Körper verbunden (unter anderem durch vermehrte Anlagerung von Körperzellen), je besser die Implantatoberfläche dem natürlichen Knochen entspricht. Dies ist das Ziel der vorliegenden Erfindung. Die chemische Zusammensetzung und vor allem auch die Oberflächenstruktur der Beschichtung bis hinab in den Mikro- und sogar Nanometerbereich soll erfindungsgemäß der natürlichen Knochenstruktur möglichst nahe kommen. Weiter soll die Beschichtung kovalent an die Oberfläche der Implantate gebunden werden. Die erfindungsgemäßen Knochenimplantatmaterialien weisen eine höhere Biokompatibilität, bessere Einheilung in den natürlichen Knochen und eine erhöhte mechanische Belastbarkeit auf.

Die Oberflächenmodifikation gemäß der vorliegenden Erfindung zielt darauf ab, knochenähnliche Strukturen kovalent gebunden auf die Oberfläche von Knochenimplantatmaterialien aufzubringen, welche sowohl die Protein- als auch die Mineralphase des natürlichen Knochens beinhalten. Dies soll die Einheilung des Implantats in den Knochen unterstützen. Diese Strukturen beinhalten eine Matrix aus denaturiertem Kollagen, der Gelatine, welche schließlich mit Calciumphosphat mineralisiert wird. Die Mineralisation erfolgt hierbei mit Hilfe von nicht-kollagenen Proteinen und deren Analoga, welche als Nukleationsinhibitoren fungieren, so dass die Mineralisation kontrolliert verläuft und eine ektopische Mineralisation vermieden wird. Solche Nukleationsinhibitoren sind beispielsweise poly-Asparaginsäure oder Fetuin. Die Mineralisation mit Octacalciumphosphat oder Hydroxylapatit erfolgt durch das Inkubieren der Gelatinematrix in einer Lösung, welche Calciumionen oder Phosphationen beinhaltet. Durch eine langsame und kontrollierte Zugabe einer Lösung der jeweils komplementären Phosphationen oder Calciumionen kann Octacalciumphosphat und/oder Hydroxylapatit innerhalb der Gelatinematrix ausgefällt werden. Durch die relativ ungeordnete Struktur der Gelatine besitzt die resultierende Oberflächenmodifikation geflechtknochenartige oder kallusartige Struktur. Die Knochenzellen könnten so bei der Einheilung des Materials weitere ungeordnete Kollagenstrukturen um das Material herum aufbauen bzw. das Material direkt weiter mit dem Knochen verknüpfen. Diese ungeordneten Strukturen können dann schließlich in der natürlichen Remodellierungsphase der Knochenwundheilung zu geordneten Knochenstrukturen umgebaut werden. Hierbei können die Zellen durch die kovalente Anbindung der Proteine bei der Remodellierung allerdings nicht bis zur direkten Oberfläche des Implantatmaterials vordringen und verbleiben so immer in einer gewünschten Matrix aus extrazellulären Proteinen. Das Implantatmaterial wird damit für die Zellen maskiert, um bei der Einheilung von Implantaten unerwünschte Reaktionen zu vermeiden. Da die Modifikationen lediglich die Oberfläche der Implantatmaterialien betrifft, werden Materialeigenschaften nicht verändert.

Die grundlegenden chemischen Reaktionen können leicht für die Modifikation verschiedener Materialien adaptiert werden. So können Metalloxidoberflächen kovalent über etablierte Silanchemie angebunden werden. Dies macht die erfindungsgemäße Oberflächenbeschichtung auch interessant für oxidische Keramikmaterialien. Da weiterhin Metalle beispielsweise durch Plasmabehandlung unschwer an der Oberfläche oxidierbar sind, werden über die Silanchemie auch die gängigen Implantatmaterialien aus Titan der erfindungsgemäßen Oberflächenmodifizierung über Silane zugänglich.

Die Figuren zeigen:
Fig. 1: Struktureller Aufbau von Kollagenfasern.

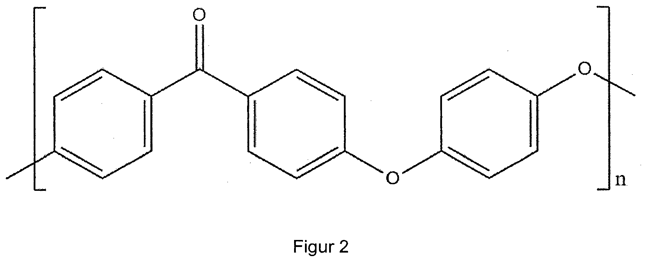

Fig. 2: Strukturformel von Polyetheretherketon (PEEK).
Fig. 3: ATR-IR-Spektrum des unmodifizierten PEEK.
Fig. 4: Übersichtsspektrum der XPS-Messung des unmodifizierten PEEK.
Fig. 5: Feinspektren des C_{1S}-Signals (A) und des O_{1S}-Signals (B) der XPS-Messung des unmodifizierten PEEK.
Fig. 6: ¹H-NMR-Spektrum des unmodifizierten PEEK.
Fig. 7: Ergebnis der rasterkraftmikroskopischen Untersuchung des unmodifizierten PEEK. A: Überblick über eine 50 µm x 50 µm Fläche; B: Höhenprofil zu Bild A; C: Überblick über eine 4 µm x 4 µm Fläche; D: Höhenprofil zu Bild C.

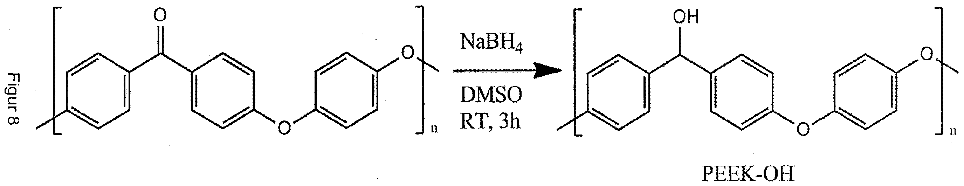

Fig. 8: Reaktionsschema der Reduktion des PEEK.
Fig. 9: ¹H-NMR-Spektrum des PEEK-OH.
Fig. 10: ATR-IR-Spektrum des PEEK-OH.
Fig. 11: Übersichtsspektrum der XPS-Messung des PEEK-OH.
Fig. 12: Feinspektren des C_{1S}-Signals (A) und des O_{1S}-Singals (B) der XPS-Messung des PEEK-OH.
Fig. 13: Theoretische Werte der C_{1S} und O_{1S}-Komponenten und deren Zuordnung zur chemischen Struktur des PEEK-OH.
Fig. 14: Ergebnisse der rasterkraftmikroskopischen Untersuchung des PEEK-OH. a: Oberflächendarstellung, b: Höhenprofil.
Fig. 15: Reaktionsschema der Ankopplung des Bersteinsäurelinkers.
Fig. 16: ATR-IR-Spektrum des PEEK-COOH.
Fig. 17: ¹H-NMR-Spektrum des PEEK-COOH.
Fig. 18: Reaktionsschema der Ankopplung des Maleimid an PEEK-OH.
Fig. 19: ATR-IR Spektrum des PEEK-OH im Vergleich zu PEEK-Maleimid.
Fig. 20: Reaktionsschema der Ankopplung des Hexamethylendiisocyanat an PEEK-OH.
Fig. 21: ATR-IR-Spektren des PEEK-OH im Vergleich zu PEEK-NCO.
Fig. 22: Übersichtsspektrum der XPS-Analyse des PEEK-NCO.
Fig. 23: Feinspektren der XPS-Messung des PEEK-NCO des O_{1S}- (A), C_{1S}- (B) und N_{1S}-Übergangs (C).
Fig. 24: Reaktionsschema der Ankopplung von Gelatine an PEEK-COOH.
Fig. 25: Ergebnis der REM- und anschließenden EDX-Untersuchung auf Stickstoff auf der Oberfläche des PEEK-Gelatine-Films.
Fig. 26: Ergebnis der REM Untersuchung an einer Kantenfläche der Oberfläche des PEEK-Gelatine-Films (a) Übersicht, (b) Nahaufnahme der Oberfläche, (c) unmodifizierte PEEK-Oberfläche als Referenz.
Fig. 27: Reaktionsschema der Ankopplung von Gelatine über einen Isocyanat-Linker.
Fig. 28: Ergebnis der REM-Untersuchung der Oberfläche des PEEK-Gelatine Films über Isocyanat-Linker.
Fig. 29: Ergebnis der REM-Untersuchung der Oberfläche des PEEK-Gelatine Films über Maleimid-Linker.
Fig. 30: Ergebnisse der REM-Untersuchung der Oberfläche des mineralisiertem PEEK-HAp Films.
Fig. 31: REM Aufnahme (a) der mit FIB geschnitten mineralisierten Folie und deren komplementäre TEM Aufnahme (b). c,d: TEM-Aufnahme der mineralisierten Grenzfläche und Elektronenbeugungsbilder an den gezeigten TEM Bildausschnitten. e: TEM-Aufnahme eines Ultramikrotomieschnittes der mineralisierten Folie. f: Elektronenbeugungsbild der Mineralschicht an der Grenzfläche.
Fig. 32: Ergebnisse des Proliferationstests der NHDF auf PEEK, PEEK-Gelatine und PEEK-HAp.
Fig. 33: MTT-Viabilitätstest der NHDF auf PEEK, PEEK-Gelatine und PEEK-HAp.
Fig. 34: Ergebnisse des Adhäsionstests der NHDF auf PEEK, PEEK-Gelatine und PEEK-HAp.

Die vorliegende Erfindung wird anhand der folgenden, nicht-einschränkenden Beispiele näher erläutert.

### Beispiel 1:

### Analyse unmodifizierten PEEK-Materials

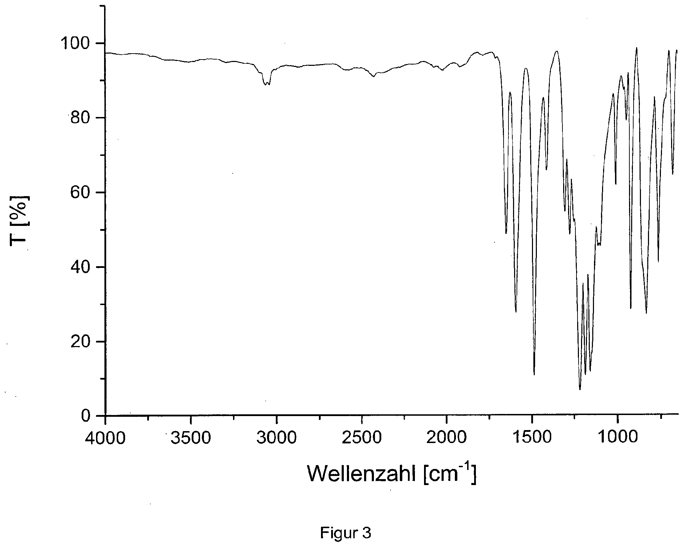

Das Material wurde als PEEK-optima von Invibio, Hofheim gekauft. Diese Folien wiesen eine amorphe Struktur auf und erschienen deshalb transparent mit einer weiß-beigen Färbung. Ein ATR-IR-Spektrum des unmodifizierten Materials ist in Figur 3 zu sehen und zeigt ein Spektrum, welches gut mit Literaturergebnissen übereinstimmt. Typische Banden für aromatische Polymere wie PEEK sind die aromatischen Streckschwingungen bei 1650 cm⁻¹, 1593 cm⁻¹ und 1486 cm⁻¹, sowie die Diarylether-Streckschwingung bei 1216 cm⁻¹. Da PEEK eine relativ starke Autofluoreszenz aufweist konnte eine komplementäre Raman-Untersuchung nicht durchgeführt werden.

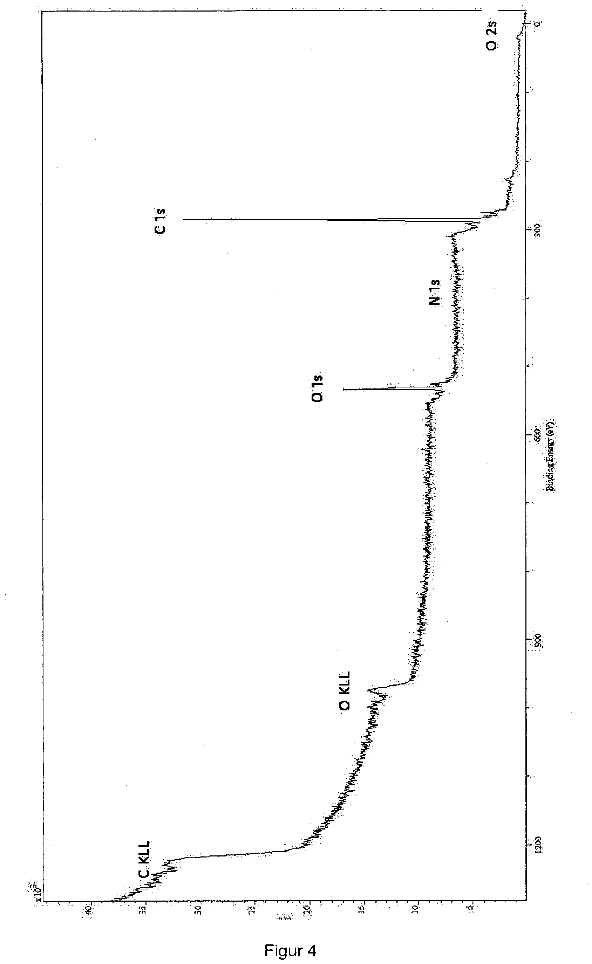

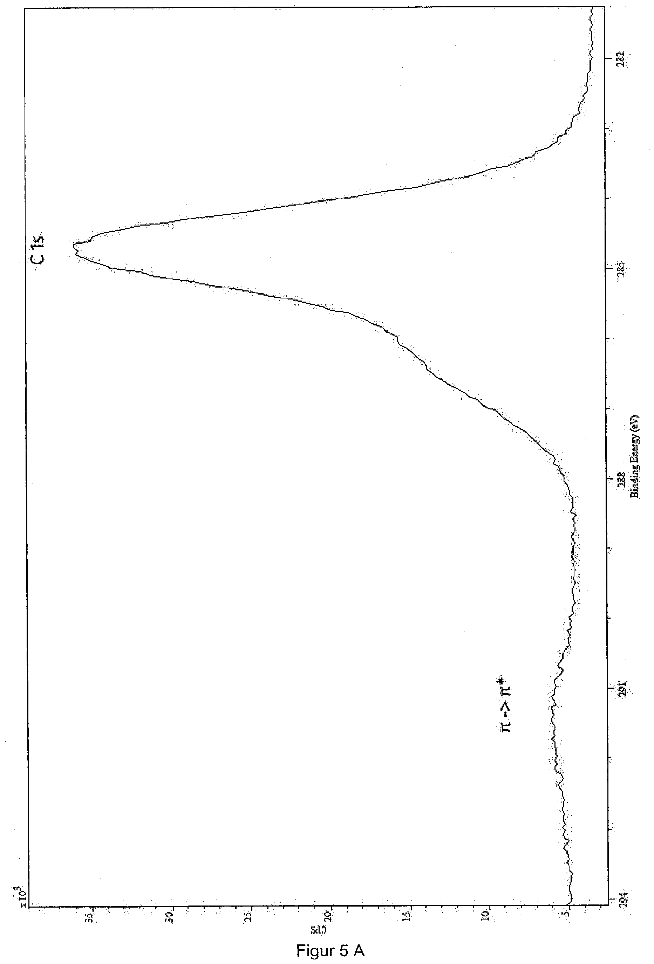

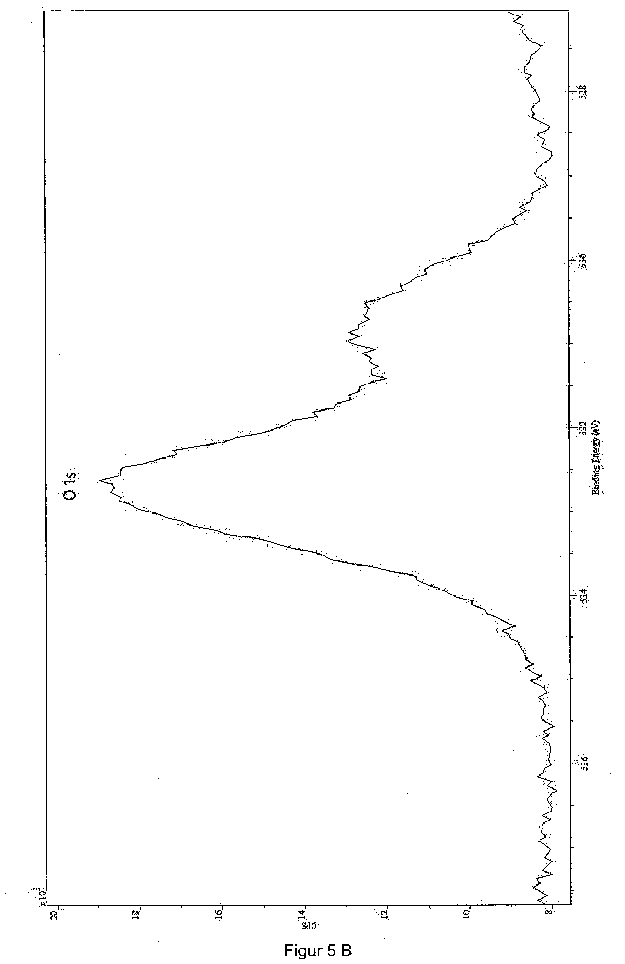

Des Weiteren wurde die Oberfläche mit Röntgenphotoelektronenspektroskopie (XPS) untersucht. Ein Übersichtsspektrum ist in Figur 4 zu sehen und die Ergebnisse sind in Tabelle 1 zusammengefasst. Da PEEK ein nichtleitendes Material ist kam es während der Messung zu Aufladungserscheinungen, welche in einer Gleichgewichtsaufladung und einer einhergehenden Verschiebung der Signale um einige Elektronenvolt führten. Dies konnte aufgrund des Fehlens einer Kompensationsmethode im apparativen Aufbau nicht minimiert werden. Kohlenstoff hat in PEEK drei unterschiedliche chemische Umgebungen, welche in drei identifizierbaren und unterscheidbaren Bindungsenergien zwischen den Atomen für den C_{1S} Übergang führen. Das C_{1S} Spektrum (Fig. 5 A) zeigt eine C-C/C-H-Komponente bei 284,7 eV, eine C-O-Komponente bei 286,3 eV, eine C=O-Komponente bei 287,1 eV und einen π-Übergangs-Satellitenpeak bei 291,4 eV. Diese haben jeweils eine relative Zusammensetzung von 75,6%, 17,7%, 3,6% und 3,1%. Sauerstoff hat in PEEK unterschiedliche chemische Umgebungen, welche in zwei nahe beieinanderliegenden Bindungsenergien resultiert. Das O_{1S}-Feinspektrum (Fig. 5 B) zeigt eine O=C-Komponente bei 531,1 eV und eine O-C-Komponente bei 533,3 eV mit einer relativen Konzentration von 26,4% und 73,6%. Die Gesamtkonzentration des Kohlenstoffs und Sauerstoffs im PEEK betrug 74,6% und 24,2%. Hiermit wurde eine C:O-Verhältnis von 3,03 berechnet. Da der theoretisch zu erwartende Wert einer stöchiometrisch zusammengesetzten PEEK-Wiederholungseinheit 6,33 ist, wird angenommen, dass sich Wasser oder andere Sauerstoff enthaltende Verbindungen während der Messung auf der Oberfläche befanden. Da das Material vor der Messung nicht explizit getrocknet wurde und unter trockenen Bedingungen in die Messkammer befördert wurde erscheint es möglich, dass Luftfeuchtigkeit an der Oberfläche des Materials adsorbierte. Andererseits wurde ebenfalls eine Stickstoff enthaltende Komponente an der Oberfläche des Materials von 1,2% festgestellt. Diese könnte von dem Herstellungsprozess des Materials oder durch Verunreinigungen während des Transportes resultieren.

**Tabelle 1: Zusammenfassung der Ergebnisse der XPS-Analyse des unmodifizierten PEEK.**

| Zuordnung | Element/ | BE [eV] | Komponentenzusammensetzung | Elementkonzentration |
|---|---|---|---|---|
| | Transition | | [Atom-%] | [Atom-%] |
| Kohlenstoff | C 1s | 284.7 | 75.6 | 74.52 |
| | C 1s | 286.3 | 17.7 | |
| | C 1s | 287.1 | 3.6 | |
| | C 1s | 291.4 | 3.1 | |
| Sauerstoff | O 1s | 531.1 | 26.4 | 24.28 |
| | O 1s | 533.3 | 73.6 | |
| Stickstoff | N 1s | | 100 | 1.2 |

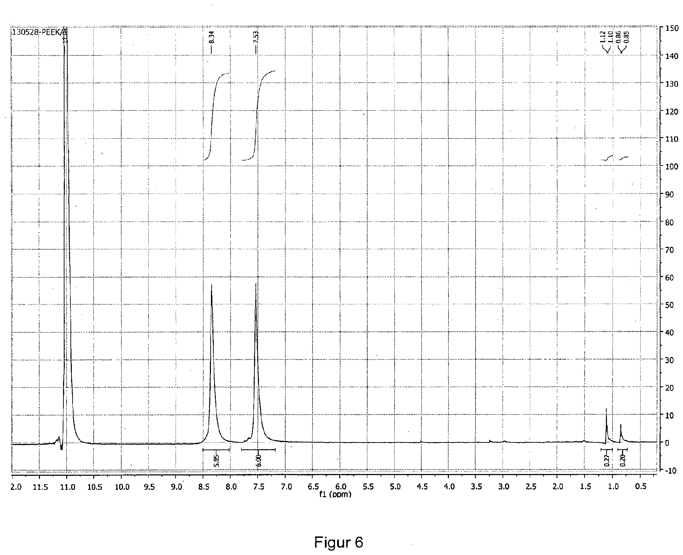

Des Weiteren wurden NMR-spektroskopische Untersuchungen mit CF₃SO₃D als Lösungsmittel durchgeführt. Das ¹H-NMR Spektrum ist in Figur 6 dargestellt und weist zwei charakteristische Peaks bei 8,34 ppm und 7,53 ppm mit jeweils der gleichen Intensität auf. Diese wurden den aromatischen Protonen zugeordnet. Weiter konnten zwei weitere Signale bei 1,11 ppm und 0,85 ppm, beide jeweils ein Dublett, gemessen werden. Diese resultieren vermutlich von aliphatischen Protonen. Dies stärkt die Vermutung von Verunreinigungen an oder im Material, da sich in reinem PEEK keine aliphatischen Protonen befinden sollten. Ein HSQC-NMR-Experiment zeigte eine Bindung der aliphatischen Protonen zu Kohlenstoffatomen, welche bei einer Verschiebung von163,4 ppm und 163,19 ppm erscheinen.

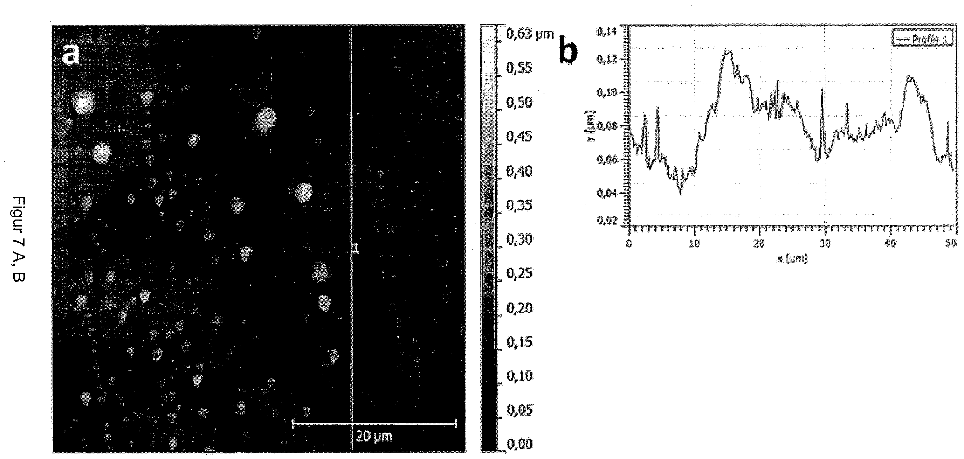

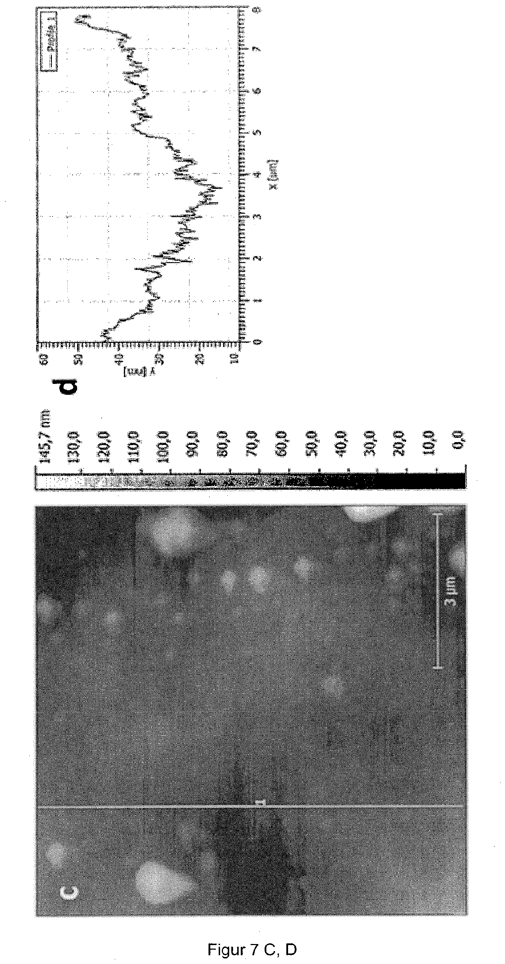

Die Oberfläche des Materials wurde weiter mit Rasterkraftmikroskopie (AFM) charakterisiert. Die Ergebnisse sind in Figur 7 dargestellt. Die Oberfläche zeigt eine relativ glatte und gleichbleibende Struktur. Es ist wahrscheinlich, dass die vereinzelten Unebenheiten auf der Oberfläche von Staubpartikeln aus der Luft resultieren, da sich das Rasterkraftmikroskop nicht in einer staubfreien Umgebung befindet. Der maximale Höhenunterschied wurde anhand eines Übersichtsbildes (Abbildung 7 B, D; Profil 2) erstellt und beträgt mit gemessenen Staubpartikeln 433 nm und ohne circa 17 bis 33 nm.

Ein wichtiger Faktor für die Erhöhung der Biokompatibilität ist eine Erhöhung der Hydrophilie der Oberfläche des Materials, da hierdurch eine hervorragende Benetzung durch das in der operativen Wunde des Knochens befindliche Blut und Körperflüssigkeit und der damit verbundenen Adhäsion der Osteogenese stimulierenden Faktoren gegeben ist. So erfolgte eine ergänzende Oberflächencharakterisierung durch Wasser-Kontaktwinkelmessungen. Der Kontaktwinkel betrug 89° ± 1°. Dies entspricht einer geringen Hydrophilie beziehungsweise einer geringen Benetzbarkeit des Materials.

### Beispiel 2:

### Nasschemische Modifikationen des PEEK - Reduktion zu PEEK-OH

Um die Biokompatibilität des Materials zu erhöhen muss die Oberfläche zunächst aktiviert werden um nachfolgend Biopolymere wie zum Beispiel Gelatine chemisch anbinden zu können.

Im Falle des PEEK-Materials wurde zunächst die Keto-Gruppe zu einer Hydroxylgruppe reduziert um für die nachfolgenden Kopplungsreaktionen als Ankerpunkt zwischen Gelatineschicht und Material zu dienen. Dies geschah nach einer im Stand der Technik bekannten Modifikation. Hierfür wurde eine PEEK-Folie in eine Lösung von Natriumborhydrid in Dimethylsulfoxid getaucht. Im Folgenden wird das entstandene Produkt PEEK-OH genannt.

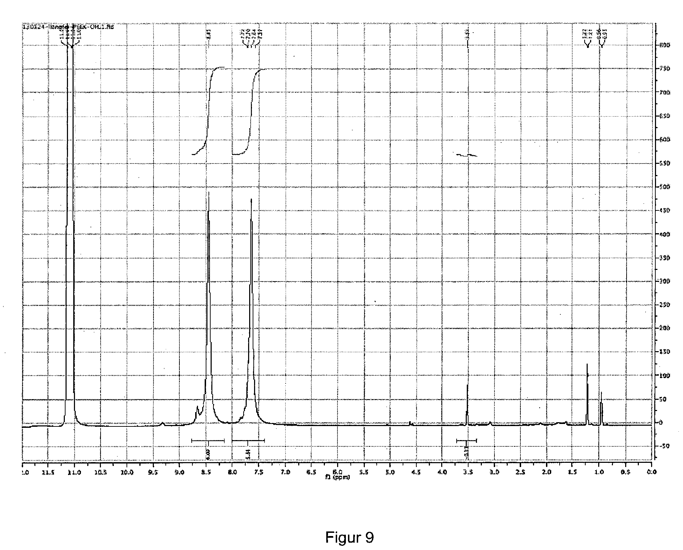

Um den Erfolg der Reaktion zu verifizieren wurde NMR-Spektroskopie verwendet (Figur 9). Da das unmodifizierte Material nur aromatische Protonen aufweist, sollte ein Erscheinen von nichtaromatischen Protonen in einem ¹H-NMR-Experiment direkt mit der Hydroxylmodifikation und damit dem Erfolg der Reaktion korrelieren.

Ebenfalls sind durch den Vorher-/Nacher-Vergleich die aliphatischen Protonen von den entstandenen Benzhydryl-Proton-Signalen gut zu unterscheiden und identifizieren. Das ¹H-NMR-Spektrum zeigt ein Singulett bei δ=8.56 ppm, welches typisch für eine carbokationische Spezies ist. Die Existenz dieser Spezies ist aufgrund der Verwendung einer sehr starken Säure als Lösungsmittel, welche das PEEK-OH dehydriert haben könnte, wenig überraschend. Das Carbokation ist aufgrund seiner Positionierung zwischen zwei aromatischen Systemen gut resonanzstabilisiert und kann so gemessen werden. Des Weiteren ist dem Spektrum ein weiteres neues Signal bei 3.41 ppm zu entnehmen, welches dem Benzhydrylproton entsprechen könnte. Die Feinaufspaltung des Signals ist ein Dublett mit einer Kopplungskonstante von 3.1 Hz. Diese könnte von der ⁴J-Kopplung von des Benzhydrylprotons zu den aromatischen Protonen stammen. Ein HSQC-NMR-Experiment zeigte eine Kopplung des carbokationischen-Proton-Signals zu einem Kohlenstoff-Signal, welches bei einer Verschiebung von 113.8 ppm erscheint. Das als Benzhydryl identifizierte Signal koppelt zu einem Kohlenstoff-Signal bei einer Verschiebung von 34,5 ppm, was einem Erfolg der Reaktion entsprechen würde.

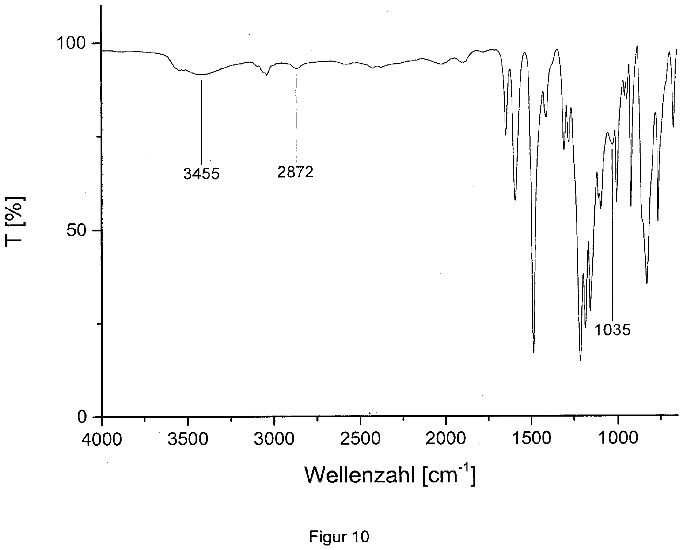

Die Oberfläche wurde weiterhin mit ATR-IR-Spektroskopie analysiert. Das Spektrum ist in Figur 10 dargestellt. Das Spektrum weist im Vergleich zum unmodifizierten Material eine breite Bande bei circa 3400 cm⁻¹ auf, welche einer OH-Streckschwingung zugeordnet wurde, eine neue Bande bei 2874 cm⁻¹, welche einer C-H-Streckschwingung zugeordnet wurde und eine neue Bande bei 1035 cm⁻¹, welche einer C-O-Streckschwingung des Alkohols zugeordnet wurde.

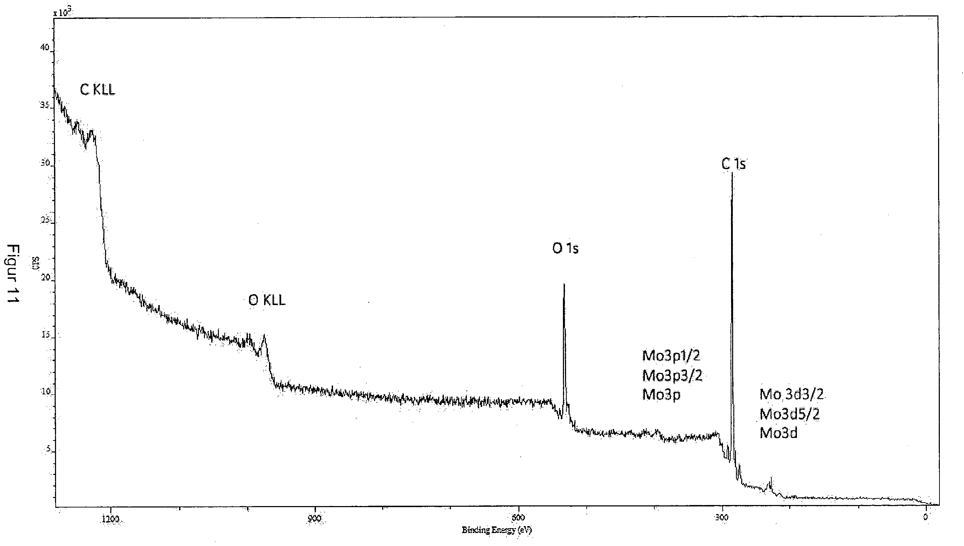

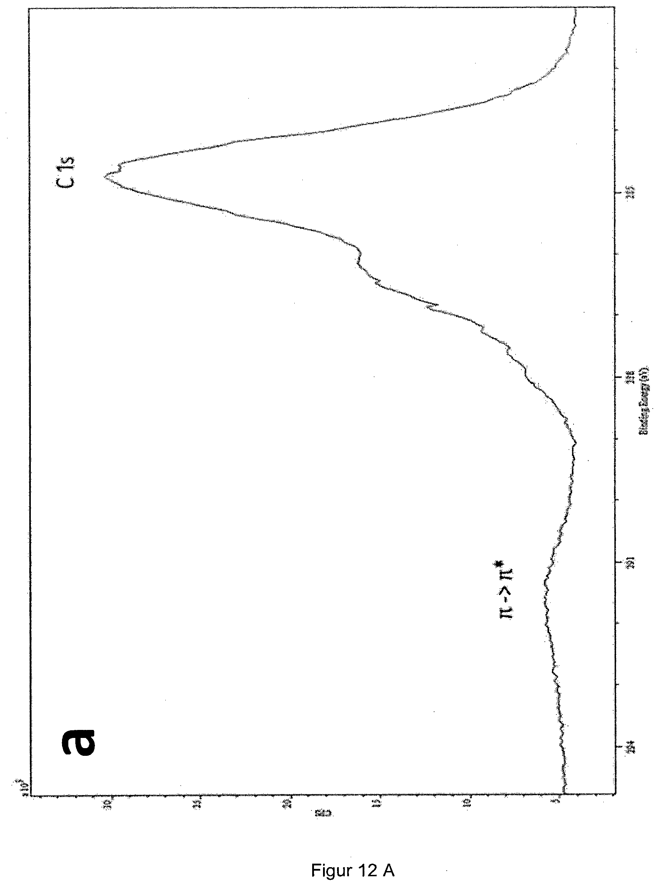

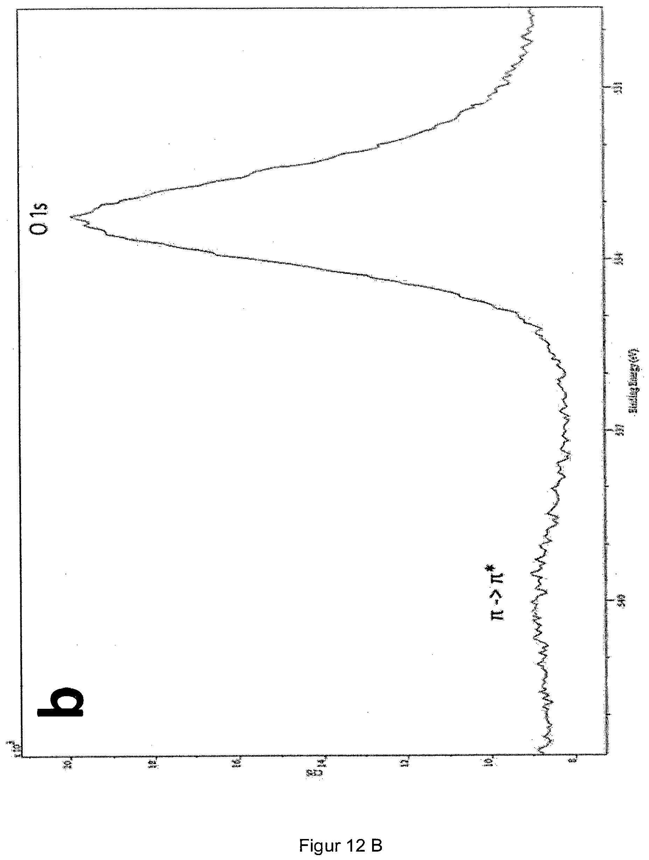

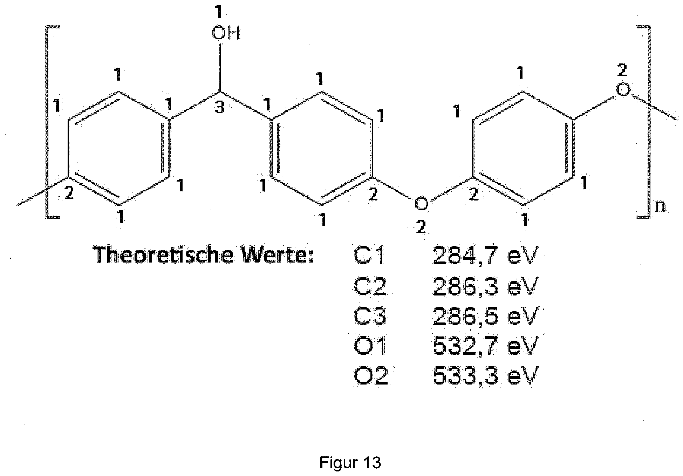

Die Oberfläche des Materials wurde weiter mit XPS analysiert und mit dem unmodifizierten Material verglichen. Das Übersichtsspektrum und die dazugehörigen Feinspektren sind in den Figuren 11 und 12 dargestellt. Um die Oberflächenaufladung während der Messung zu bestimmen und zu kompensieren, wurde eine Molybdän-Referenz verwendet. Die Ergebnisse sind in Tabelle 2 zusammengefasst. Die Fits wurden anhand der theoretischen Werten von relevanten Kohlenstoff und Sauerstoff-Bindungen von Polymeren erstellt. Das C_{1S} Feinspektrum zeigt eine C-C/C-H-Komponente bei 284.7 eV, eine C-O-Komponente bei 286.3 eV, eine C=O-Komponente bei 286.5 eV und ein π-Übergang Satelliten-Signal bei 291.5 eV. Diese Signale zeigen eine relative Zusammensetzung von 63.07%, 32.25%, 2.34% und 3.32%. Somit konnte eine Verminderung der C=O-Komponente und eine damit einhergehende Erhöhung der C-O-Komponente gemessen werden. Das O_{1S} Feinspektrum zeigt eine O=C-Komponente bei 531.1 eV, eine O-C-Komponente bei 532.2 eV, eine O-H-Komponente bei 533.3 eV und ein π-Übergang Satelliten-Signal bei 539.8 eV. Diese haben eine relative Zusammensetzung von 11.1%, 32.2%, 51.4% und 5.3%. Aus diesen Werten ergab sich eine C:O-Rate von 3,16. Da der theoretisch berechnete Wert für eine stöchiometrisch zusammengesetzte Wiederholeinheit von PEEK 6,33 beträgt, wird davon ausgegangen, dass nicht 100% der Keto-Gruppen zu Hydroxylgruppen reduziert wurden oder sich wiederum Verunreinigungen aus den bereits oben genannten Quellen auf der Oberfläche befinden. Theoretische Werte der C_{1S}- und O_{1S}-Komponenten und deren Zuordnung zur chemischen Struktur von PEEK-OH sind in Figur 13 gezeigt.

**Tabelle 2: Zusammenfassung der XPS-Analyse des PEEK-OH.**

| Zuordnung | Element/ | BE [eV] | Komponentenzusammensetzung | Elementkonzentration |
|---|---|---|---|---|
| | Übergang | | [Atom-%] | [Atom-%] |
| Kohlenstoff | C 1s | 284.7 | 63.09 | 68.4 |
| | C 1s | 286.3 | 32.25 | |
| | C 1s | 286.5 | 2.34 | |
| | C 1s | 291.5 | 2.32 | |
| Sauerstoff | O 1s | 531.1 | 11.1 | 31.6 |
| | O 1s | 532.2 | 32.2 | |
| | O 1s | 533.3 | 51.4 | |
| | O 1s | 539.8 | 5.3 | |

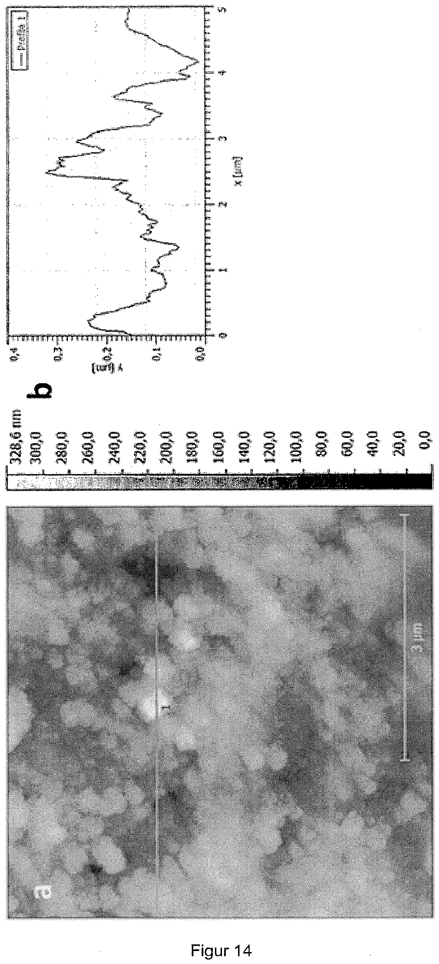

Die Oberfläche des PEEK-OH-Materials wurde weiterhin mit Rasterkraftmikroskopie untersucht. Vor der Reaktion betrug die maximale Höhendifferenz 17 bis 33 nm in einer 16 µm² Analysefläche. Nach der Reaktion betrug die maximale Höhendifferenz 208 nm (Figur 14). Diese relativ große Höhendifferenz führte zu weiteren Komplikationen für die weitere Nutzung dieser Methode für die Analyse des Materials. Wenn im nächsten Schritt Gelatine an der Oberfläche angekoppelt wird, könnte der Höhenunterschied und die Struktur nicht ausgeprägt genug sein um die Gelatineschicht vom Bulkmaterial unterscheiden zu können. Aus diesem Grund wurden auf weitere AFM-Messungen im Folgenden verzichtet.

Messungen der Wasserkontaktwinkel zeigten eine Abnahme des Kontaktwinkels von zuvor 89° des unmodifizierten Materials zu 77° des modifizierten Materials, was eine Zunahme der Hydrophilie der Oberfläche bedeutet.

Wegen der starken Autofluoreszenz des PEEK-Materials konnten keine Fluoreszenzfarbstoffe als indirekter Nachweis und für eine eventuelle Quantifizierung herangezogen werden.

### Beispiel 3:

### Ankopplung von Linker-Molekülen - PEEK-COOH

Um die kovalente Anbindung der Gelatinemoleküle an die PEEK-OH Oberfläche zu gewährleisten, muss diese mit entsprechenden Linkermolekülen chemisch modifiziert werden. Dazu bieten sich nachstehend näher bezeichnete Reaktionen an. Im Falle von Metalloxidoberflächen würden Silane als Linkermoleküle zum Einsatz kommen.

Um eine Oberfläche mit Säuregruppen zu erzeugen, wurde an die Oberfläche ein Linkermolekül über eine Esterbindung angebunden. Dieses sollte eine Dicarbonsäure sein, welche durch Methylengruppen variabler Anzahl untereinander verbunden ist. Exemplarisch wurde die Reaktion an einem Bernsteinsäure-Linker durchgeführt.

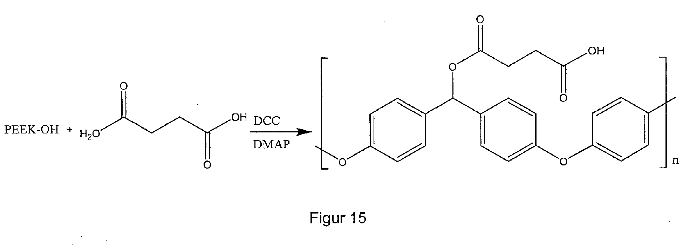

Dies wurde über Zuhilfenahme von N,N'-Dicyclohexylcarbodiimid (DCC) und 4-dimethylaminopyridin (DMAP) realisiert. Typischerweise wurden bei der Reaktionsdurchführung zwei Äquivalente Bernsteinsäure zunächst in Tetrahydrofuran (THF) gelöst und mit einem Eisbad gekühlt. Dann wurde ein Äquivalent DCC und 0.1 Äquivalent DMAP in die gekühlte Lösung zugegeben. Nach vier Stunden wurden die PEEK-OH Folien in diese Lösung zugegeben und für weitere vier Tage bei Raumtemperatur gerührt. Hiernach wurden die Folien drei Mal mit THF und drei Mal mit Aceton gewaschen. Die Trocknung erfolgte schließlich in einem Vakuumofen bei maximal 40°C und 50 mbar. Das Reaktionsschema der Ankopplung des Bernsteinsäurelinkers ist in Figur 15 gezeigt.

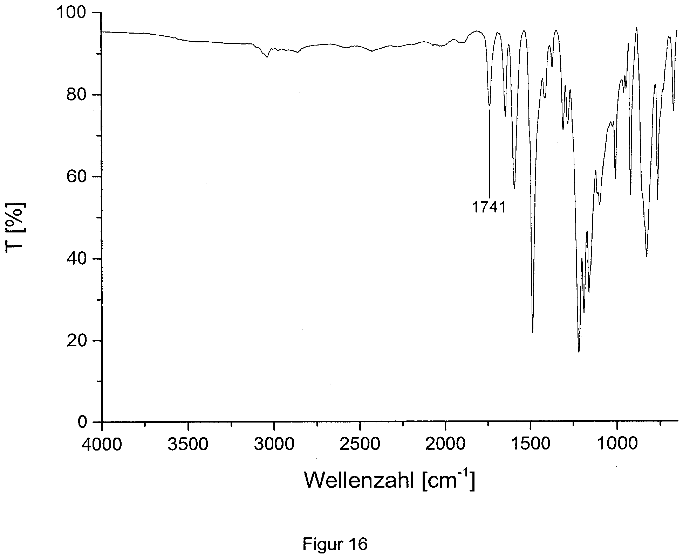

Die Folien wurden mit ATR-IR-Spektroskopie untersucht (Figur 16). In den Spektren konnte eine neue Bande bei 1741 cm⁻¹ ausgemacht werden, welche charakteristisch für eine Ester-Gruppe ist und somit den Erfolg der Reaktion indiziert.

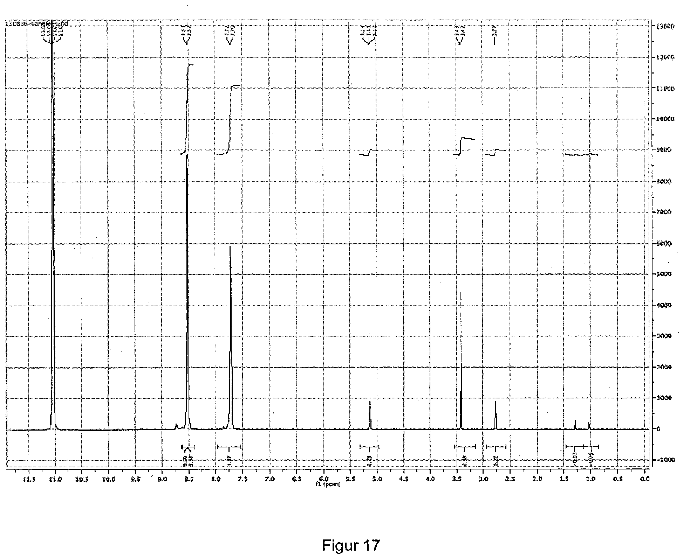

Des Weiteren wurde NMR-Spektroskopie verwendet, um den Erfolg der Reaktion zu verifizieren (Figur 17). In einem ¹H-NMR-Experiment zeigten sich im Vergleich zum Spektrum der Vorstufe zwei neue nicht-aromatische Triplett-Signale bei 2.8 ppm und 5.2 ppm mit identischen Integrationskorrelationen.

Weiter wurden auch an diesem Produkt Kontaktwinkelmessungen durchgeführt. Diese zeigten eine weitere Abnahme des Wasserkontaktwinkels von zuvor 77° der Vorstufe zu 70° des PEEK-COOH. Dieses Ergebnis indiziert eine weitere Zunahme der Hydrophilie.

### Beispiel 4:

### Ankopplung von Linker-Molekülen - PEEK-Maleimid

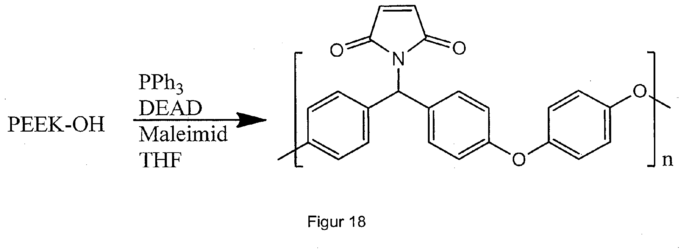

Kopplungsreagenzien, welche Bindungen mit Thiolgruppen eingehen können, wie beispielsweise Maleimid-Linker, sind weit verbreitet unter den Protein- und anderen Biokonjugations-Techniken. Thiolgruppen sind in Proteinen häufig an Disulfidbindungen beteiligt, wobei die Vernetzung über solche Gruppen die Proteinstruktur nur unwesentlich verändert. Thiolgruppen kommen ebenfalls in den meisten Proteinen vor, sind aber nicht so zahlreich wie primäre Amine und machen die Kopplungsreaktion so wesentlich selektiver. Ein weiterer Vorteil einer Thioether-Bindung ist deren Irreversibilität. Für die Anbindung solcher Moleküle wird häufig die Mitsunobu-Reaktion herangezogen. Das Reaktionsschema der Ankopplung des Maleimid an PEEK-OH ist in Figur 18 gezeigt.

Hierzu wurde die PEEK-OH-Folie in eine Lösung aus Triphenylphosphan, Azodicarbonsäurediethylester und Maleimid in THF getaucht und für 24h bei Raumtemperatur gerührt. Danach wurde der Film vier Mal mit einer Lösung aus Ether/Hexan (1:1) gewaschen nachfolgend in einem Vakuumofen für mindestens drei Stunden bei 60°C und 50 mbar getrocknet. Im Folgenden wird das Produkt PEEK-Maleimid genannt.

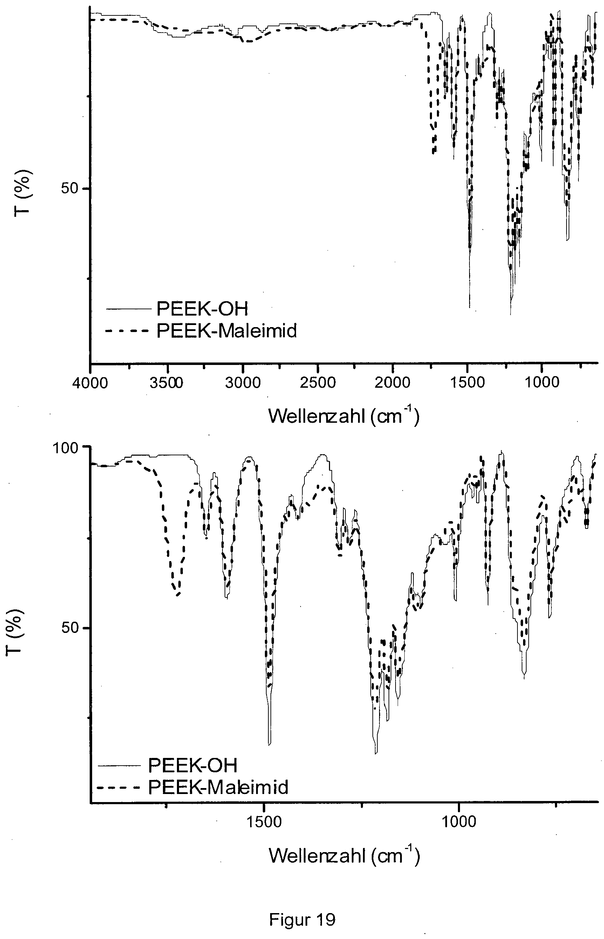

Nachfolgend wurde die PEEK-Maleimid-Folie mit ATR-IR-Spektroskopie untersucht. Im Spektrum (Figur 19) ist die Entstehung neuer Banden bei 2870 cm⁻¹ und 2936 cm⁻¹ zu erkennen, welche der C-H-Strecksschwingung zugeordnet wurden. Weitere neue Signale sind bei 1789 cm⁻¹ und 1717 cm⁻¹, welche den symmetrischen Carbonyl-Streckschwingungen zugeordnet wurden, 1440 cm⁻¹, welches der symmetrischen C-N-C-Schwingung zugeordnet wurde und bei 722 cm⁻¹ und 693 cm⁻¹, welches der Ringtorsion zugeordnet wurde.

Eine Analyse mit energiedispersiver Röntgenspektroskopie (EDX) zeigt die Anwesenheit von Stickstoff auf der Oberfläche. Bei fast neutralen Reaktionsbedingungen (pH-Wert 6,5 bis 7,5) bilden sich bevorzugt stabile Thioether-Bindungen. Bei basischeren Reaktionsbedingungen (pH > 8,5) binden bevorzugt primäre Amine, wobei aber gleichzeitig die Hydrolyserate der Maleimid-Gruppe zu einer unreaktiven Maleamidsäure zunimmt.

### Beispiel 5:

### Ankopplung von Linker-Molekülen - PEEK-NCO

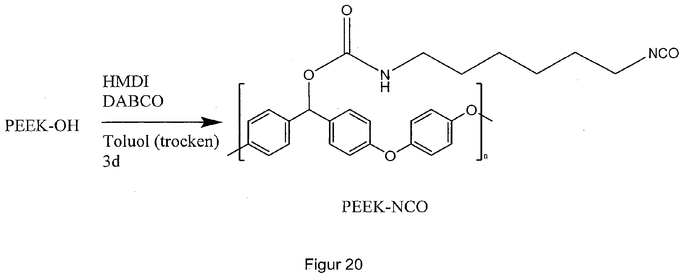

In einem parallelen Ansatz wurde ein homobifunktioneller Hexamethylendiisocyanat-linker (HMDI-Linker) verwendet. Dieser kann unter anderem mit Aminen stabile Isourea-Bindungen eingehen. Solche Isocyanat-Linker sind ebenfalls dazu in der Lage, Moleküle, welche eine Hydroxylgruppe beinhalten, wie beispielsweise Polysaccharide, in einer Carbamat/Urethanbindung zu koppeln. Für die Modifikation des PEEK-OH wurde die PEEK-OH Folie in eine Lösung aus Hexamethylendiisocyanat und katalytischen Mengen von 1,4-Diazabicyclo[2.2.2]octan (DABCO) in Toluol getaucht und für drei Tage bei Raumtemperatur gerührt. Die Reaktion wurde unter Schutzatmosphäre unter trockenen Reaktionsbedingungen durchgeführt. Nach der Reaktionszeit wurde die Folie zwei Mal mit Toluol und mit Aceton gewaschen und schließlich in einem Vakuumofen für mindestens drei Stunden bei 30°C und 50 mbar getrocknet. Im Folgenden wird das entstandene Produkt PEEK-NCO genannt. Das Reaktionsschema der Ankopplung von Hexamethylendiisocyanat an PEEK-OH ist in Figur 20 gezeigt.

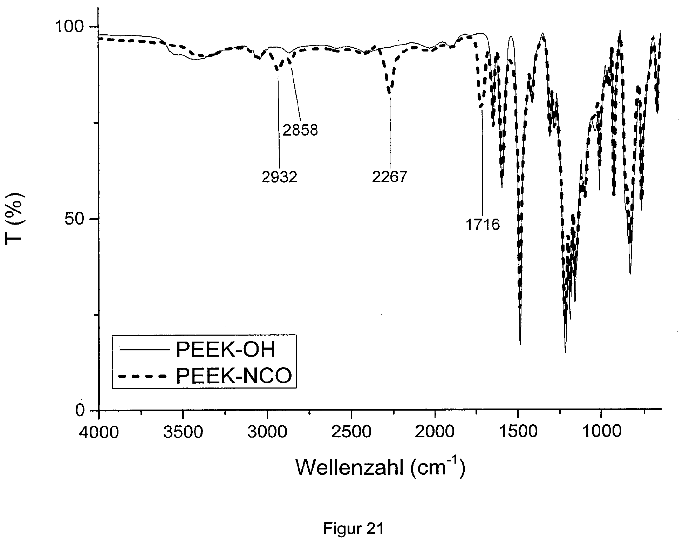

Nach der Reaktion wurden die PEEK-NCO-Folien mit ATR-IR-Spektroskopie untersucht. Das Spektrum (Figur 21) zeigt eine neue Bande bei 2267 cm⁻¹, welche der Isocyanat-Gruppe zugeordnet wurde, zwei weitere Banden bei 2932 cm⁻¹ und 2858 cm⁻¹, welche der CH-Streckschwingung des Linkers zugeordnet wurden, und eine Bande bei 1716 cm⁻¹, welche der Carbonylstreckschwingung des Amids zugeordnet wurde. Die zwei zu beobachteten Banden bei ca. 3420 cm⁻¹ und ca. 3348 cm⁻¹ stammen vermutlich von einer partiellen Reduktion der Isocyanatgruppe aufgrund der Anwesenheit von Luftfeuchtigkeit.

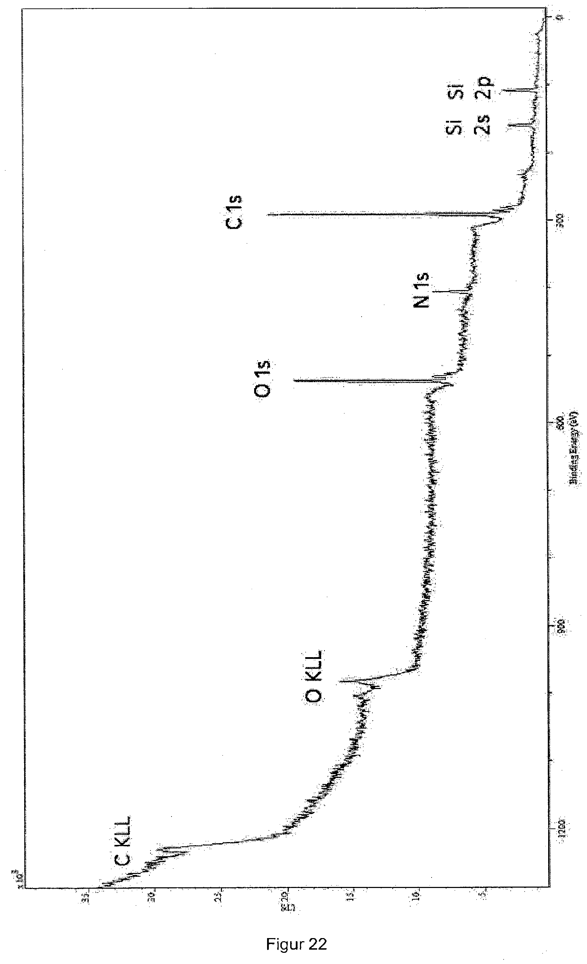

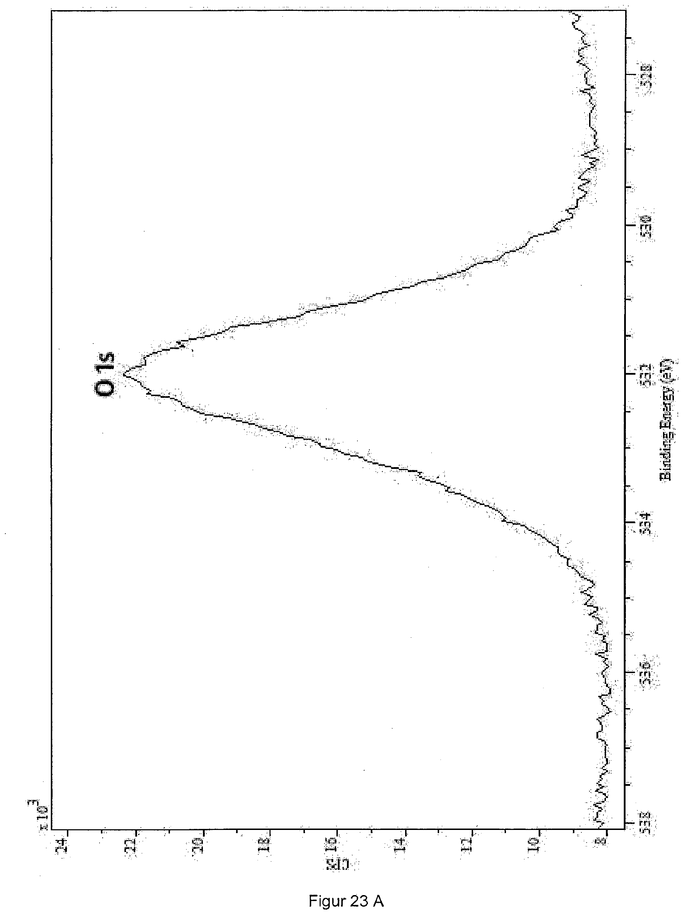

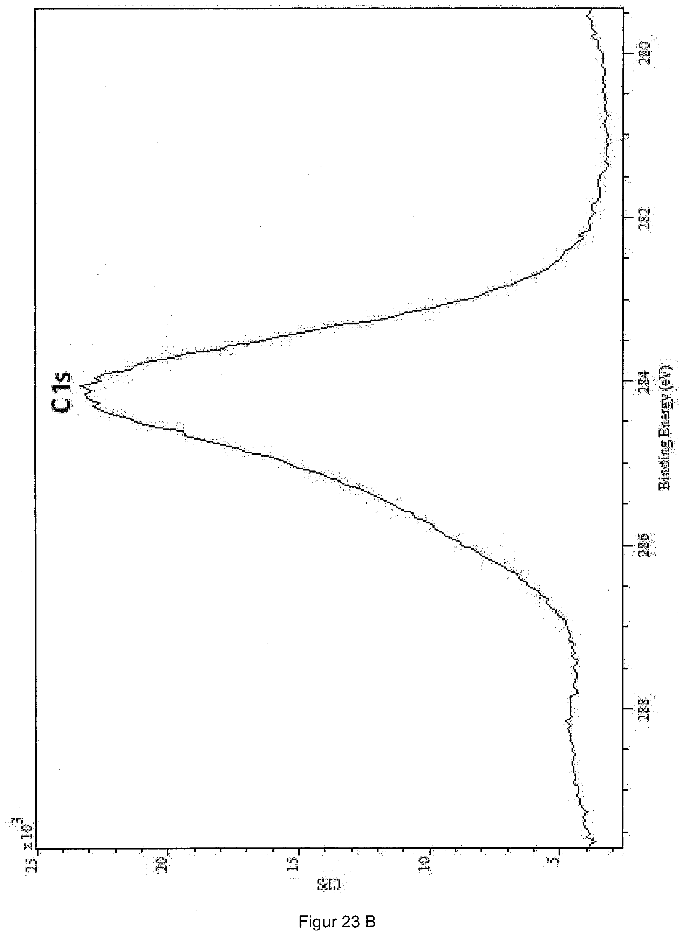

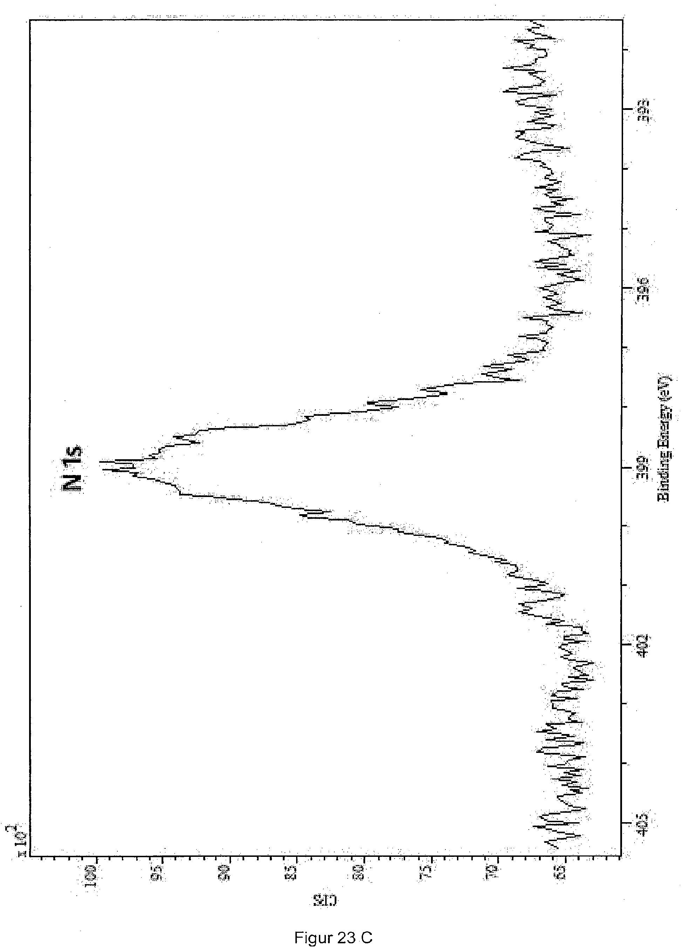

Die Oberflächen des Materials wurden weiter mit XPS untersucht und mit dem unmodifizierten Material verglichen. Als Referenzmaterial wurde hier Silizium verwendet. Das Übersichtsspektrum ist in Figur 22 gezeigt. Es zeigt ein Signal bei 399,1 eV und zeigt so die Anwesenheit von Stickstoff an der Oberfläche. Die Feinspektren der C_{1S}-, O_{1S}- und N_{1S}-Komponenten sind in Figur 23 dargestellt. Die Ergebnisse der Fits sind in Tabelle 3 zusammengefasst. Die Feinstruktur der N_{1S}-Komponente zeigt die Anwesenheit von Stickstoff in zwei unterschiedlichen chemischen Umgebungen bei 399.9 eV und 399.0 eV mit einer relativen Intensität von 30% zu 70%. Eine quantitative Analyse zeigte einen Gesamtstickstoffgehalt von 9,2% im Analysevolumen. Der Kohlenstoffanteil war 54,92%. Durch Fitting des Feinspektrums des C_{1S}-Signals konnten einzelne chemische Umgebungen der Kohlenstoffe identifiziert werden. Diese zeigten Signale bei 284,7 eV, welche der C-C/C-H-Komponente zugeordnet wurden, 285,2 eV, welche der CH₂-Komponente zugeordnet wurden, 287,0 eV, welche der C=O-Komponente des Amides zugeordnet wurden, und 289,3 eV, welche der C=O-Komponente des NCO zugeordnet wurden. Die jeweiligen relativen Konzentrationen waren 39,1%, 53,2%, 1,35%, 2,85%. Dabei konnte eine weitere Komponente bei 286,5 eV identifiziert werden. Ob diese von der C-O-, C-OH- oder C=O-Komponente (unmodifiziertes PEEK) stammte, konnte nicht näher bestimmt werden.

Der Sauerstoffgehalt betrug 35,6% mit einer O_{1S}-Komponente bei 532,0 eV. Durch Fitten des Feinspektrums konnte Sauerstoff in drei unterschiedlichen chemischen Umgebungen identifiziert werden. Ein Signal bei 531,1 eV, welche der O=C-Komponente zugeordnet wurde, ein Signal bei 532,6 eV, welche der O-H-Komponente zugeordnet wurde, und ein Signal bei 533,3 eV, welche der O-C-Komponente zugeordnet wurde. Diese Signale haben eine relative Intensität von 22,6%, 19,9% und 57,5%. Bei dieser Auswertung muss allerdings beachtet werden, dass der HMDI-Linker viele Nebenreaktionen eingehen kann, die sich bei dieser Art Auswertung problematisch auswirken können. Beispielsweise können die HMDI-Linker untereinander unter Bildung einer Allophanatgruppe ein Dimer oder gar Multimer an der Oberfläche bilden. Da über XPS nur die obersten paar Nanometer analysiert werden, ist es schwierig die exakte Komponentenkonzentration nach einer solchen Polymerisierung der Linker abzuschätzen.

**Tabelle 3: Zusammenfassung der XPS-Messung des PEEK-NCO.**

| Zuordnung | Element/ | BE [eV] | Komponentenzusammensetzung | Elementkonzentration |
|---|---|---|---|---|
| | Übergang | | [Atom-%] | [Atom-%] |
| Kohlenstoff | C 1s | 284.7 | 39.1 | 54.92 |
| | C 1s | 285.2 | 53.2 | |
| | C 1s | 287.0 | 1.35 | |
| | C 1s | 289.3 | 2.85 | |
| | C 1s | 285.5 | 2.4 | |
| | C 1s | 286.5 | 1.06 | |
| Sauerstoff | O 1s | 531.1 | 22.6 | 35.6 |
| | O 1s | 532.6 | 19.9 | |
| | O 1s | 533.3 | 57.5 | |
| Stickstoff | N 1s | 399.9 | 30 | 9.2 |
| | N 1s | 399.0 | 70 | |
| Silizium | Si 2s | 149 | | 0.28 |

Aufgrund der fehlenden Hydrolysebeständigkeit der HMDI-Linker gegenüber Wasser wurde auf eine Kontaktwinkelmessung verzichtet.

Rasterelektronenmikroskopie zeigt eine relativ glatte, homogene Oberfläche. Eine Analyse mit EDX bestätigte die Anwesenheit von Stickstoff auf der Oberfläche. Die Stickstoffkonzentration betrug hier 7,67%.

### Beispiel 6:

### Ankopplung der Biomoleküle - Kopplung über EDC

Um den Reaktionsverlauf zu testen und für die Optimierung der Reaktionsbedingungen wurden in Hinblick auf die höheren Kosten des Kollagens die Gelatine als zu koppelndes Biomolekül gewählt, da Gelatine im Wesentlichen denaturiertes Kollagen darstellt.

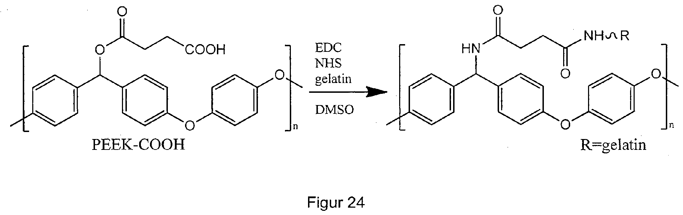

Die Ankopplung der Gelatine geschah unter Zuhilfenahme von 1-Ethyl-3-(3-dimethylaminopropyl)carbodiimid Hydrochlorid (EDC). Dies ist ein weitverbreitetes und kommerziell erhältliches Kopplungsreagenz, welches häufig für die chemische Kopplung von Proteinen und Peptiden, Proteinen und Oligonukleotiden oder Biomolekül und Partikeloberflächen angewendet wird. Zusammen mit N-Hydroxysuccinimid (NHS) wird im Speziellen eine Reaktion von Carboxylaten und Aminen unter Bildung einer Amidbindung gefördert. EDC-Kopplungsreaktionen werden üblicherweise unter sauren Reaktionsbedingungen (pH 4.5 bis 5.5) durchgeführt. Das Reaktionsschema der Ankopplung von Gelatine an PEEK-COOH ist in Figur 24 gezeigt.

Um eine einfache Kopplung der Gelatinemoleküle untereinander zu verhindern, wurde zunächst die Oberfläche des PEEK-COOH mit der EDC/NHS-Lösung aktiviert und die Filme schließlich von dieser Lösung in eine Gelatinelösung transferiert und weitere vier Stunden gerührt. Anschließend wurde die Folie mindestens drei Mal mit ca. 40°C warmen Wasser gewaschen, um adsorbierte Gelatine abzuwaschen. Schließlich wurde die Folie mit Ethanol gewaschen und getrocknet, welches gleichzeitig die restlichen Gelatinemoleküle auf der Oberfläche präzipitiert. Die entstandene Folie wird im Folgenden PEEK-Gelatine genannt.

Die PEEK-Gelatine-Folien wurden schließlich mit ATR-IR-Spektroskopie untersucht. Hier konnten allerdings keine signifikanten Unterschiede im Spektrum im Vergleich zur Vorstufe festgestellt werden. Dies liegt vermutlich an dem Überlapp der sehr starken charakteristischen Banden für das PEEK Material und der im Vergleich schwächeren Banden der Amidbanden, welche ebenfalls in demselben Wellenzahlenbereich erscheinen müssten.

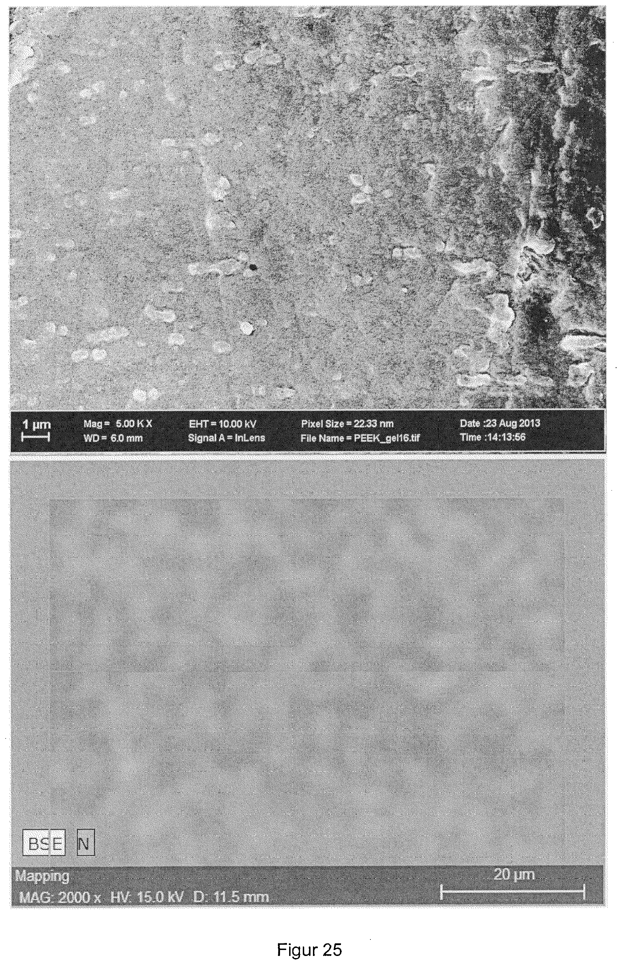

Um den Erfolg der Reaktion der Reaktion dennoch zu verifizieren wurden weitere Analytikmethoden herangezogen. Die Oberfläche wurde weiter mit Rasterelektronenmikroskopie untersucht. Das Bild (Figur 25) zeigt homogen verteilte, ungeordnete Strukturen auf der Oberfläche der Folie, welche nicht auf dem unmodifizierten Material oder der Vorstufe zu finden waren. Da die Folie zuvor mehrmals mit warmen Wasser gewaschen wurde, sollte sich keine adsorbierte Gelatine an der Oberfläche befinden und alle sichtbaren Strukturen auf der Oberfläche kovalent angebundene Gelatine sein. Eine direkt anschließende EDX-Analyse zeigt die Anwesenheit von Stickstoff auf der Oberfläche. Der Stickstoff kann hierbei nur von der angekoppelten Gelatine herrühren.

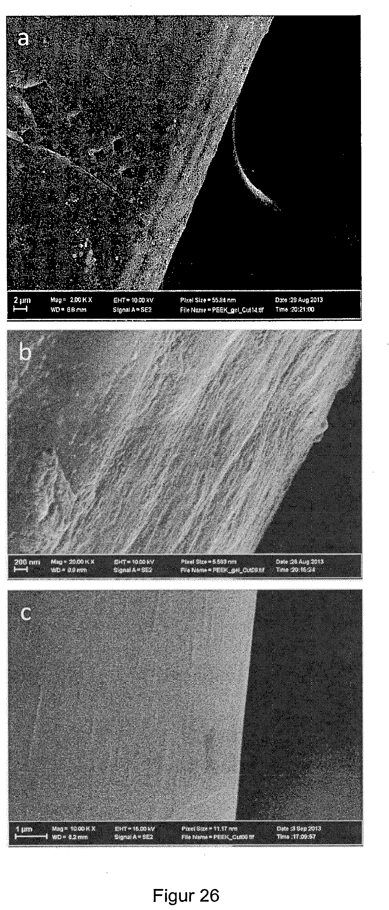

Um einen Eindruck der Dicke der Gelatineschicht zu bekommen, wurde der PEEK-Gelatine Film geschnitten und die Kantenfläche mittels Rasterelektronenmikroskopie untersucht (Figur 26). Diese Untersuchung ergab, dass die Gelatineschicht durchschnittlich 100 bis 150 nm dick zu sein scheint.

Eine thermogravimetrische Untersuchung ergab einen Massenanteil von 25 Gewichtsprozent Gelatine in der Analysensubstanz.

### Beispiel 7:

### Ankopplung der Biomoleküle - Kopplung über Isocyanat-Linker

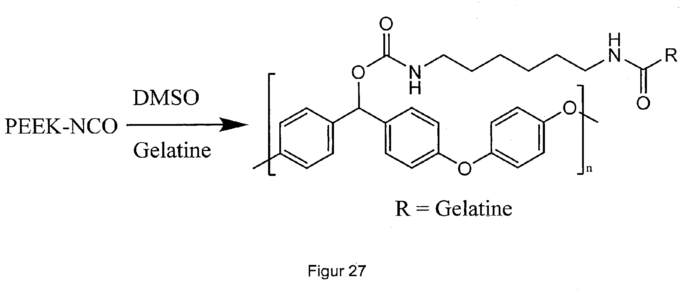

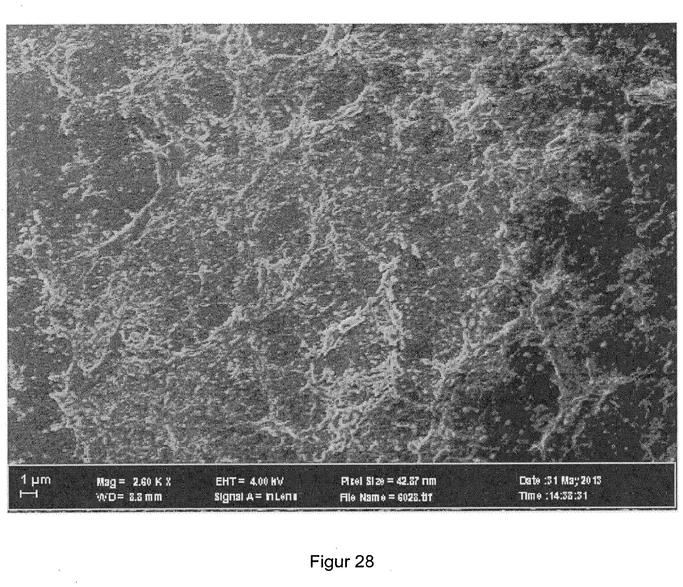

Die Ankopplung der Gelatine an die Oberfläche des PEEK-NCO erfolgte aus einer Lösung von Gelatine in DMSO. Das Reaktionsschema der Ankopplung von Gelatine über einen Isocyanat-Linker ist in Figur 27 gezeigt. Die Oberfläche wurde auch hier mit Rasterelektronenmikroskopie untersucht. Das Bild (Figur 28) zeigt homogen verteilte, ungeordnete Strukturen auf der Oberfläche der Folie, welche nicht auf dem unmodifizierten Material oder der Vorstufe zu finden waren. Da die Folie zuvor mehrmals mit warmen Wasser gewaschen wurde, sollte sich auch hier keine adsorbierte Gelatine an der Oberfläche befinden und alle zu sehenden Strukturen auf der Oberfläche kovalent angebundene Gelatine sein. Eine komplementäre EDX-Analyse konnte hierbei nicht für eine Verifizierung herangezogen werden, da sich allein durch den Isocyanat-Linker Stickstoff an der Oberfläche befindet.

ATR-IR spektroskopische Untersuchungen ergaben im vorher/nachher-Vergleich ein Verschwinden des charakteristischen Isocyanat-Signals bei 2267 cm⁻¹, was ein Indiz für eine erfolgte Gelatine-Kopplung ist.

### Beispiel 8:

### Ankopplung der Biomoleküle - Kopplung über Maleimid-Linker

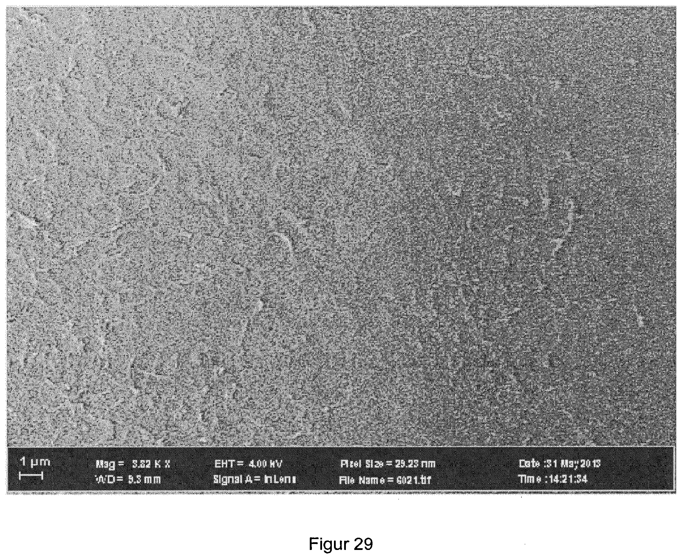

Die Ankopplung der Gelatine an die Oberfläche des PEEK-Maleimid erfolgte aus einer Lösung von Gelatine in Wasser. Hierfür wurde die PEEK-Maleimid-Folie in einer 3% Lösung von Gelatine in Wasser für 3 h bei Raumtemperatur gerührt. Anschließend wurde die Folie drei Mal mit warmen Wasser, und schließlich ein Mal mit Aceton gewaschen. Die Trocknung des Filmes erfolge auch hier in einem Vakuumofen für mindestens drei Stunden bei maximal 40°C und 50 mbar. Die Oberfläche wurde auch hier mit Rasterelektronenmikroskopie untersucht. Das Bild (Figur 29) zeigt ungeordnete Strukturen auf der Oberfläche der Folie, welche nicht auf dem unmodifizierten Material oder der Vorstufe zu finden waren. Da die Folie zuvor mehrmals mit warmen Wasser gewaschen wurde, sollte sich auch hier keine adsorbierte Gelatine an der Oberfläche befinden und alle sichtbaren Strukturen auf der Oberfläche kovalent angebundene Gelatine sein. Eine komplementäre EDX-Analyse konnte auch hier nicht für eine Verifizierung herangezogen werden, da sich allein durch den Maleimid-Linker Stickstoff an der Oberfläche befindet.

ATR-IR spektroskopische Untersuchungen ergaben im vorher/nachher-Vergleich ein Verschwinden des Signals bei 2870 cm⁻¹, was ein Indiz für eine erfolgte Gelatine-Kopplung ist.

### Beispiel 9:

### Biomineralisation

Um die kovalent angekoppelte Gelatineschicht schließlich zu mineralisieren, wurde die PEEK-Gelatine-Folie in eine 2-(4-(2-Hydroxyethyl)-1-piperazinyl)ethansulfonsäure (HEPES)-Pufferlösung, welche Calciumchlorid, Dikaliumhydrogenphosphat und poly-Asparaginsäure enthält, für vier Tage bei 37°C getaucht.

Die Folien wurden schließlich mindestens drei Mal mit Wasser und drei Mal mit Ethanol gewaschen. Die Trocknung des Materials wurde in einem Vakuumofen (maximal 40°C bei 50 mbar) durchgeführt. Das entstandene Material wird im Folgenden PEEK-HAp genannt.

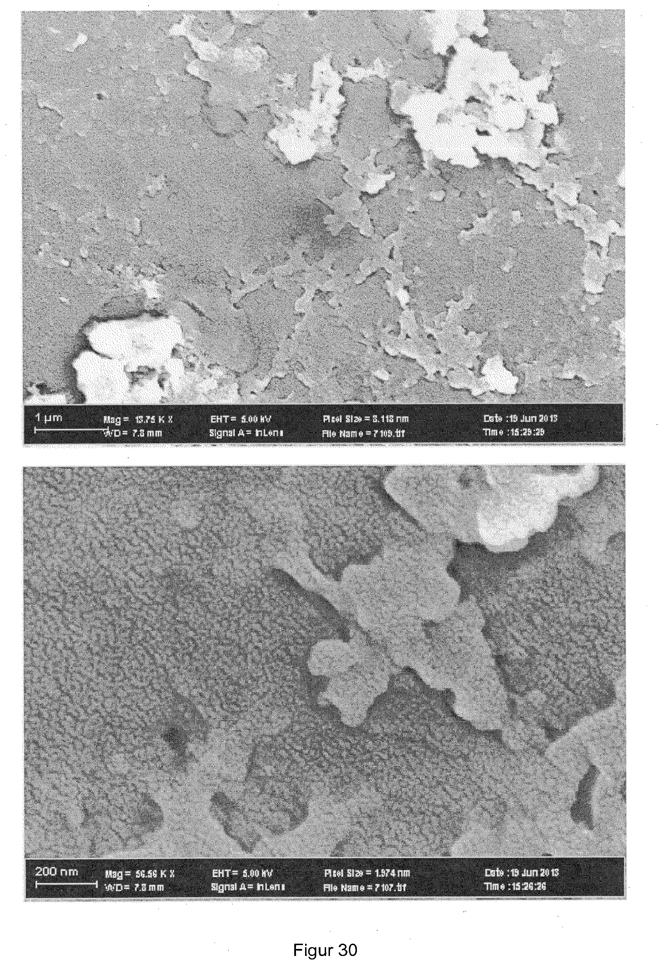

Die Oberfläche wurde mit Rasterelektronenmikroskopie untersucht (Figur 30). Dabei konnten auf der Oberfläche einige große und zufällig verteilte Strukturen ausgemacht werden, welche aus der Oberfläche zu wachsen scheinen. Zwischen diesen großen Strukturen konnten kleinere Strukturen mit ähnlicher Struktur zu den Größeren ausgemacht werden. Eine direkt anschließende EDX-Analyse zeigte, dass diese Strukturen, sowie die gesamte Oberfläche, Calcium- und Phosphor-Komponenten enthielten. Die Ergebnisse sind in Tabelle 4 zusammengefasst. Da die Oberfläche eine stärkere Rauheit aufweist, sind diese Ergebnisse eher qualitativer Natur und bestätigen, dass die gefundenen Strukturen und die Oberfläche aus Calciumphosphat aufgebaut sind. Für eine Quantifizierung mittels EDX muss die Oberfläche glatt poliert werden oder eine weitere Methode, wie beispielsweise Thermogravimetrie herangezogen werden.

**Tabelle 4: Zusammenfassung der EDX-Ergebnisse der mineralisierten PEEK-Folie**

| Element | AN | Serie | Massenprozent (norm) [wt-%] | Atomprozent [at-%] |
|---|---|---|---|---|
| Kohlenstoff | 6 | K-Serie | 77.48 | 82.45 |
| Sauerstoff | 8 | K-Serie | 21.52 | 17.19 |
| Calcium | 20 | K-Serie | 0.64 | 0.2 |
| Phosphor | 15 | K-Serie | 0.23 | 0.1 |
| Schwefel | 16 | K-Serie | 0.14 | 0.05 |

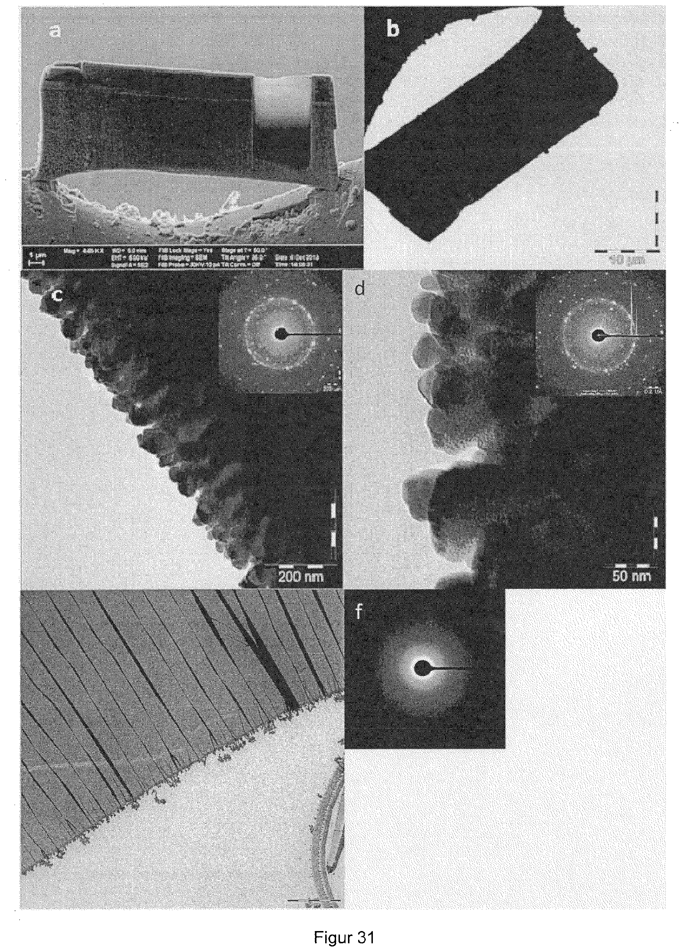

Um diese Calciumphosphatschicht zu identifizieren, wurde ein dünner Schnitt mittels eines FIB-Schnittes (Figur 31, a-d) oder Ultramikrotomschnitte (Figur 31 e) durch das Material hergestellt und schließlich mit Transmissionselektronenmikroskopie untersucht. Es ist eine dünne Schicht, welcher vermutlich die Gelatine/Apatit-Oberflächenmodifikation entspricht, direkt an der Oberfläche des PEEK Materials zu erkennen. Eine Untersuchung dieser Strukturen mittels Elektronenbeugung zeigte, dass die kristalline Phase dieser Schicht vermutlich aus amorphem Calciumphosphat besteht. Die kristallinen Signale der Beugungsbilder in Figur 31, c und d konnten eindeutig Gold zugeordnet werden, welches als Leitermaterial für die Rasterelektronenmikroskopie bzw. den FIB-Schnitt auf die Probe aufgebracht werden musste. Es konnten in diesem Beispiel keine weiteren kristallinen Signale aus diesen Beugungsbildern entnommen werden, welche die Vermutung einer amorphen Mineralphase bekräftigen. Aus Beugungsbildern von Aufnahmen von Ultramikrotomschnitten des Materials (Figur 31 e, f) konnte der amorphe Charakter der Mineralphase bestätigt werden. Auch bei den TEM-Aufnahmen der Mikrotomschnitte ist die Oberflächenmodifikation des PEEK-Materials mit einer Gelatine/Calciumphosphat-Schicht gut zu erkennen. Die Streifen im Material selber (Figur 31, e, links oben) stammen von der Auffaltung des sehr dünnen Schnittes des Materials während des Schneidevorgangs mit dem Ultramikrotom.

### Beispiel 10:

### In vitro Evaluierung der Materialien

Um die Biokompatibilität zu untersuchen, wurden die PEEK-HAp-Folien schließlich diversen *in vitro* Tests unterworfen. Diese Tests wurden gemäß der EN ISO 10993-5:2009 Richtlinie zur biologischen Evaluierung von Medizinprodukten, im Speziellen Teil 5 für die Beurteilung der *in vitro*-Zytotoxizität von Biomaterialien, durchgeführt. Alle Tests wurden in angemessenen statistischen Replikaten durchgeführt.

Zunächst sollte durch eine indirekte toxikologische Untersuchung herausgefunden werden, ob sich toxische Substanzen von dem Material ab- oder herauslösen lassen. Für die Prüfung der Extrakte wurde das Material für 24 h bei 37°C in Zellkulturmedium geschwenkt. Das Zellkulturmedium wurde schließlich unverdünnt und in Verdünnungen von 1:2, 1:4 und 1:10 in Zellkulturmedium mit L-929 Fibroblasten (P14) für zwei Tage inkubiert. Anschließend wurde der Grad der Zellzerstörung mittels Lichtmikroskopie und die Zellviabilität mittels eines MTT-Assays analysiert. Die Anwendbarkeit dieses Tests wurde mit Hilfe einer Positiv- und Negativkontrolle bestätigt. Die Lichtmikroskopie zeigte, dass nicht mehr als 10% der Zellen auf PEEK-Gelatine-Filmen und PEEK-HAp-Filmen eine runde Form aufwiesen und sich keine diskreten intrazellulären Granula in den Zellen befanden, was im gesamten einer Nichtreaktivität entspricht. Ebenfalls konnten keine Zelllyse oder leere Flächen festgestellt werden. Der MTT-Test ergab eine Zellviabilität von über 90 %. Somit können die Materialien als nicht toxisch angesehen werden.

Komplementär wurde eine direkte toxikologische Untersuchung an dem PEEK- und PEEK-HAp-Material durchgeführt. Hierfür wurden NIH 3T3 Fibroblasten für 24 h direkt auf dem Material inkubiert und hiernach mittels eines MTT-Assays die relative Zellviabilität festgestellt. Eine Lichtmikroskopische Evaluierung konnte aufgrund der Undurchsichtigkeit des Materials nur bedingt durchgeführt werden. Die Anwendbarkeit dieses Tests wurde ebenfalls durch die Anwendung einer Positivkontrolle bestätigt. Mit der lichtmikroskopischen Untersuchung konnten keine Zelllyse oder leere Flächen festgestellt werden. Der MTT-Test ergab eine Zellviabilität der Zellen von über 95% ± 2,9. Das Material kann somit als nicht toxisch angesehen werden.

Nach den toxikologischen Untersuchungen wurde das relative Proliferations-, Viabiliäts- und Adhäsionsverhalten von humanen Fibroblasten (NHDF - "normal human dermal fibroblast") einer niedrigen Passagenzahl auf dem modifizierten Material im Vergleich zum unmodifizierten Material untersucht.

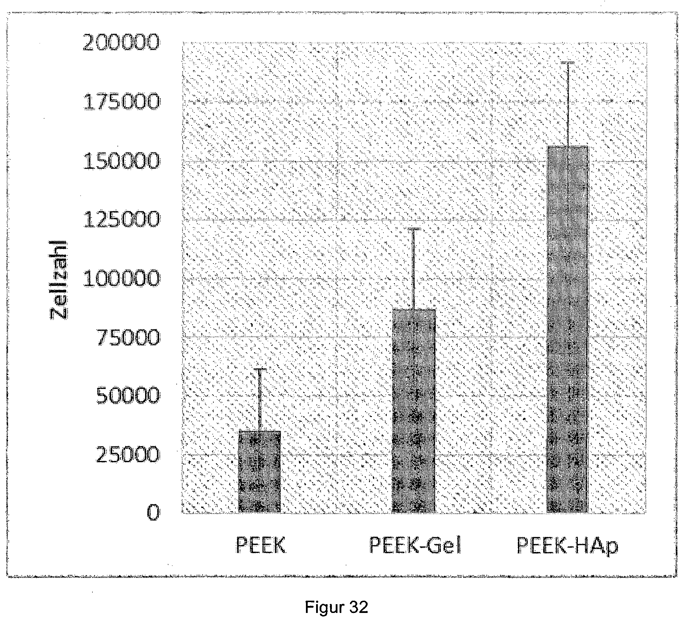

Für die Proliferation wurde eine bestimmte Anzahl an Zellen auf der Oberfläche der Materialien für 24 h inkubiert. Hiernach wurden die Zellen wieder vom Material gelöst und deren Anzahl mittels eines CASY-Zählgerätes bestimmt. Diese Untersuchung ergab, dass die Zellen auf dem PEEK-HAp-Material um einen Faktor von 4,4 besser proliferieren als auf dem unmodifizierten PEEK. Gleichzeitig wurde die Proliferation auf der Zwischenstufe des PEEK-Gelatine-Materials untersucht. Diese ergab, dass die Fibroblasten um einen Faktor 2,5 besser als auf dem unmodifizierten Material proliferieren. Somit ergibt sich, dass die Fibroblasten besser auf dem PEEK-Gelatine-Material und noch besser auf dem PEEK-HAp-Material proliferieren. Die Ergebnisse sind in Figur 32 dargestellt.

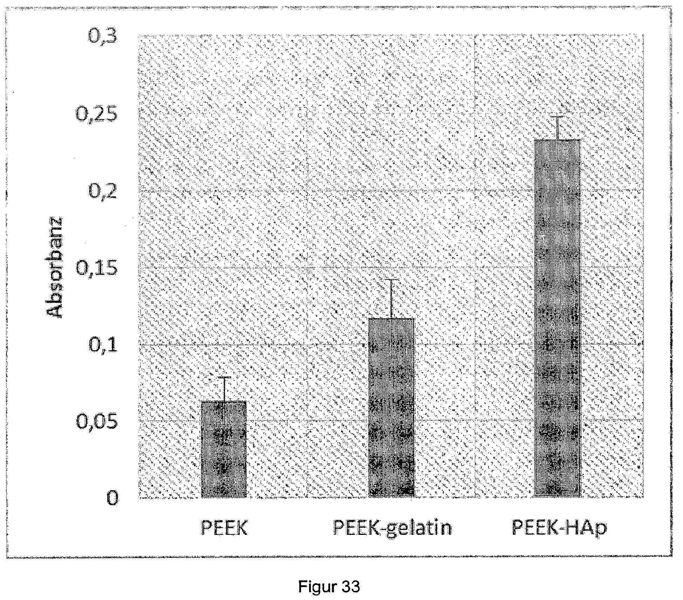

Komplementär wurde die Zellviabilität mittels eines MTT-Assays untersucht. Diese Untersuchung ergab, dass die Zellviabilität um einen Faktor von 1,8 höher auf dem PEEK-Gelatine-Material, bezogen auf das unmodifizierte PEEK-Material, und einen Faktor von 3,9 höher auf dem PEEK-HAp-Material ist. Da die Zellviabilität in direkter Korrelation mit der Zellzahl steht, ist dieser Test auch gleichzeitig eine Kontrolle für den Proliferationstest. Auch dieser Test zeigt, dass die Fibroblasten besser auf dem PEEK-Gelatine-Material und noch besser auf dem PEEK-HAp-Material proliferieren als auf dem unmodifizierten PEEK. Die Ergebnisse sind in Figur 33 dargestellt.

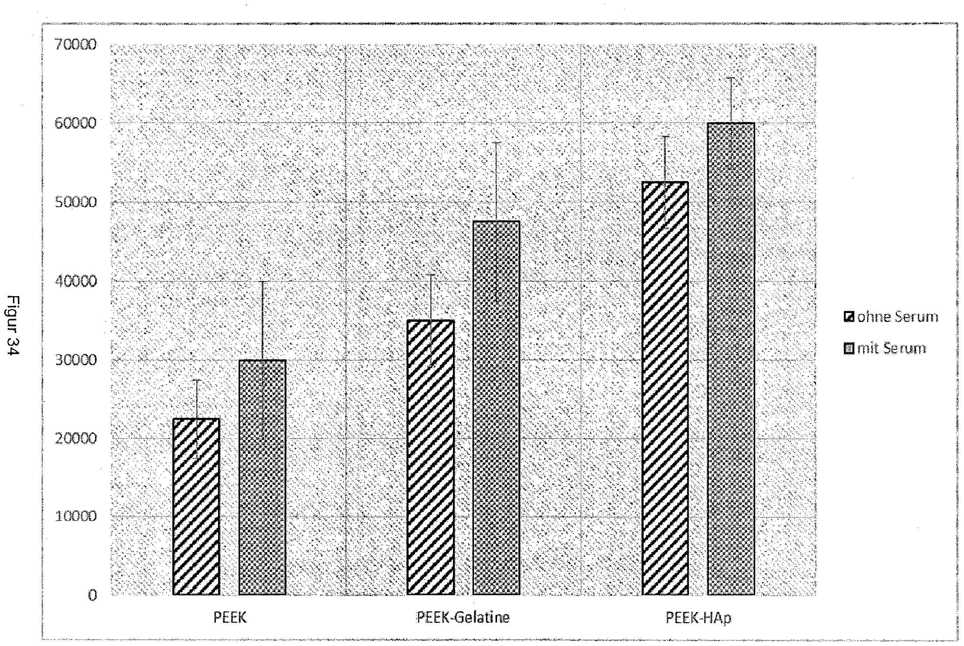

Die Zelladhäsion der Fibroblasten auf den Materialien wurde ebenfalls untersucht. Hierfür wurde eine bestimmte Anzahl der Zellen auf den Materialien ausgesät und für eine für Fibroblasten typische Adhäsionszeit auf dem Material inkubiert. Nach dieser Zeit wurden alle nicht-adhärenten Zellen abgewaschen. Bei diesem Schritt ist insbesondere auf eine reproduzierbare Waschrate zu achten. Nach diesem Waschschritt wurden die adhärenten Zellen von Material abgelöst und deren Anzahl mittels einer Neubauer-Zählkammer bestimmt. Diese Untersuchung ergab, dass die Fibroblasten in Abwesenheit von Zellkulturserum auf dem PEEK-Gelatine-Material um einen Faktor 1,5 und auf dem PEEK-HAp-Material um einen Faktor 2,3 besser adhärieren als auf dem unmodifizierten PEEK. Dieser Assay wurde sowohl in Anwesenheit als auch in Abwesenheit von Serum im Zellkulturmedium durchgeführt. Dies basiert auf der Grundlage, dass untersucht werden muss, ob die veränderte Adhäsion der Zellen aufgrund der Proteine im Zellkulturmedium, welche an der Materialoberfläche adsorbieren, oder aufgrund der vorgenommenen Oberflächenmodifikation geschieht. Die Anwesenheit von Zellkulturserum erhöht die Adhäsion der Fibroblasten um durchschnittlich 28%. Die Ergebnisse sind in Figur 34 dargestellt.

## Patentansprüche

1. Material für Knochenimplantate, umfassend:
(a) eine Oberfläche, umfassend ein Material, ausgewählt aus der Gruppe, bestehend aus oxidischen Keramikmaterialien und Polyetheretherketon (PEEK),
(b) eine kovalent an diese Oberfläche gebundene Matrix, umfassend Kollagen und/oder Gelatine, und
(c) in diese Matrix eingelagertes Calciumphosphat.

2. Material für Knochenimplantate nach Anspruch 1, wobei die kovalent an die Oberfläche gebundene Matrix Gelatine umfasst.

3. Material für Knochenimplantate nach Anspruch 1 oder Anspruch 2, wobei das Calciumphosphat ausgewählt ist aus der Gruppe, bestehend aus Calciumorthophosphat in allen mineralischen Formen.

4. Material für Knochenimplantate nach Anspruch 3, wobei das Calciumphosphat ausgewählt ist aus der Gruppe, bestehend aus amorphem Calciumorthophosphat (ACP), Dicalciumphosphat-Dihydrat (DCPD; Brushit), Octacalciumphosphat und Hydroxylapatit, auch mit partieller Fluorid-, Chlorid- oder Carbonatsubstitution, und Kombinationen davon.

5. Material für Knochenimplantate nach Anspruch 4, wobei das Calciumphosphat aus der Gruppe, bestehend aus ACP, Octacalciumphosphat und Hydroxylapatit ausgewählt ist.

6. Material für Knochenimplantate nach einem der Ansprüche 1 bis 5, wobei die Oberfläche PEEK umfasst und das Kollagen und/oder die Gelatine über einen Linker, ausgewählt aus der Gruppe, bestehend aus einem Dicarbonsäure-Linker, einem Maleimid-Linker und einem Hexamethylendiisocyanat-Linker, an das PEEK gebunden ist.

7. Material für Knochenimplantate nach einem der Ansprüche 1 bis 6, umfassend:
(a) eine Oberfläche, bestehend aus PEEK,
(b) eine kovalent an diese Oberfläche gebundene Matrix, bestehend aus Gelatine, und
(c) in diese Matrix eingelagerten Hydroxylapatit.

8. Material für Knochenimplantate nach einem der Ansprüche 1 bis 7, wobei die Matrix die gesamte Oberfläche des Materials bedeckt.

9. Verfahren zur Herstellung eines Materials für Knochenimplantate nach einem der Ansprüche 1 bis 8, umfassend die Schritte:
(a) Bereitstellen einer Oberfläche, umfassend ein Material, ausgewählt aus der Gruppe, bestehend aus oxidischen Keramikmaterialien und Polyetheretherketon (PEEK),
(b) kovalentes Ankoppeln einer Matrix, umfassend Kollagen und/oder Gelatine, an diese Oberfläche, und
(c) Mineralisieren der Matrix mit Calciumphosphat.

10. Knochenimplantat, umfassend ein festes Material und/oder einen festen Körper, auf das/den das Knochenimplantatmaterial nach einem der Ansprüche 1 bis 8 aufgebracht ist.

## Claims

1. A material for bone implants comprising:
(a) a surface comprising a material selected from the group consisting of oxide ceramic materials and polyether ether ketone (PEEK),
(b) a matrix covalently bound to said surface, comprising collagen and/or gelatin; and
(c) calcium phosphate embedded into said matrix.

2. The material for bone implants according to claim 1, wherein the matrix covalently bound to the surface comprises gelatin.

3. The material for bone implants according to claim 1 or claim 2, wherein the calcium phosphate is selected from the group consisting of calcium orthophosphate in all mineral forms.

4. The material for bone implants according to claim 3, wherein the calcium phosphate is selected from the group consisting of amorphous calcium orthophosphate (ACP), dicalcium phosphate dihydrate (DCPD; brushite), octacalcium phosphate, and hydroxyapatite, also with partial fluoride, chloride or carbonate substitution, and combinations thereof.

5. The material for bone implants according to claim 4, wherein the calcium phosphate is selected from the group consisting of ACP, octacalcium phosphate and hydroxyapatite.

6. The material for bone implants according to any one of claims 1 to 5, wherein the surface comprises PEEK and the collagen and/or gelatin is bound to the PEEK via a linker selected from the group consisting of a dicarboxylic acid linker, a maleimide linker, and a hexamethylene diisocyanate linker.

7. The material for bone implants according to any one of claims 1 to 6, comprising:
(a) a surface consisting of PEEK,
(b) a matrix covalently bound to said surface, consisting of gelatin, and
(c) hydroxyapatite embedded into said matrix.

8. The material for bone implants according to any one of claims 1 to 7, wherein the matrix covers the entire surface of the material.

9. A method for producing a material for bone implants according to any one of claims 1 to 8, comprising the steps of:
(a) providing a surface comprising a material selected from the group consisting of oxide ceramic materials and polyether ether ketone (PEEK),
(b) covalently coupling a matrix, comprising collagen and/or gelatin, to said surface, and
(c) mineralizing the matrix with calcium phosphate.

10. A bone implant comprising a solid material and/or a solid body, to which the bone implant material according to any one of claims 1 to 8 is applied.

## Revendications

1. Matériau pour implants osseux, comprenant
a. une surface comportant un matériau choisi parmi le groupe constitué par les matériaux céramiques oxidés et les polyétheréthercétones (PEEK),
b. une matrice liée de façon covalente à cette surface, comprenant du collagène et/ou de la gélatine, et
c. du phosphate de calcium inséré dans cette matrice.

2. Matériau pour implants osseux selon la revendication 1 dans lequel la matrice liée de façon covalente à la surface comprend de la gélatine.

3. Matériau pour implants osseux selon la revendication 1 dans lequel le phosphate de calcium est choisi parmi le groupe constitué par l'orthophosphate de calcium sous toutes les formes minérales.

4. Matériau pour implants osseux selon la revendication 3 dans lequel le phosphate de calcium est choisi parmi le groupe constitué par l'orthophosphate de calcium amorphe (ACP), le phosphate dicalcique dihydraté (DCPD ; Brushit), le phosphate octacalcique et l'hydroxylapatite.

5. Matériau pour implants osseux selon la revendication 4 dans lequel le phosphate de calcium est choisi parmi le groupe constitué par l'ACP, le phosphate octacalcique et l'hydroxylapatite.

6. Matériau pour implants osseux selon l'une des revendication 1 à 5 dans lequel la surface comprend du PEEK et le collagène et/ou la gélatine est liéé au PEEK par l'intermédiaire d'un groupement de liaison choisi parmi le groupe constitué par les groupements de liaison à base d'acide dicarboxylique, les groupements de liaison à base de maléimide et les groupements de liaison à base d'hexaméthylènediisocyanate.

7. Matériau pour implants osseux selon l'une des revendication 1 à 6 comportant :
(a) une surface constituée de PEEK,
(b) une matrice qui y est liée de façon covalente, constituée de gélatine, et
(c) de l'hydroxylapatite inséré dans cette matrice.

8. Matériau pour implants osseux selon l'une des revendication 1 à 7 dans lequel la matrice recouvre la totalité de la surface du matériau.

9. Procédé pour la production d'un matériau pour implants osseux selon l'une des revendications 1 à 8, comprenant les étapes:
(a) mise à disposition d'une surface comportant un matériau choisi parmi le groupe constitué par les matériaux céramiques oxidés et les polyétheréthercétones (PEEK),
(b) couplage covalent à cette surface d'une matrice comprenant du collagène et/ou de la gélatine, et
(c) minéralisation de la matrice avec du phosphate de calcium.

10. Implant osseux comprenant un matériau solide et/ou un corps solide sur lequel est appliqué le matériau pour implant osseux selon l'une des revendications 1 à 8.
